# EUROPEAN PATENT SPECIFICATION

(11) **EP 4 604 864 B1**
(45) Date of publication and mention of the grant of the patent: **25.11.2026**
(21) Application number: 23808952.8
(22) Date of filing: 19.10.2023
(51) Int. Cl.: A61B 18/14, A61B 18/00, A61M 25/00

(54) **CATHETER SHAFT HAVING HIGHLY RESILIENT RUBBER MEMBER AND METHOD OF MANUFACTURE**
KATHETERSCHAFT MIT HOCHELASTISCHEM GUMMIELEMENT UND HERSTELLUNGSVERFAHREN
TIGE DE CATHÉTER AYANT UN ÉLÉMENT EN CAOUTCHOUC HAUTEMENT ÉLASTIQUE ET PROCÉDÉ DE FABRICATION

(30) Priority: 20.10.2022 US 202263417925 P
(43) Date of publication of application: 27.08.2025
(73) Proprietor: St. Jude Medical, Cardiology Division, Inc., St. Paul, MN 55117 (US)
(72) Inventor: GUO, Xiaoping, Saint Paul, Minnesota 55117 (US)
(74) Representative: Mathys & Squire
(86) International application number: PCT/US2023/035534
(87) International publication number: WO 2024/086297

(56) References cited:
- US-A- 5 855 552
- US-A- 6 032 061
- US-A1- 2013 150 808
- US-A1- 2014 261 985

## Description

### FIELD OF DISCLOSURE

This disclosure relates generally to an elongate catheter-based cardiovascular medical device and related components. More particularly, this disclosure relates to an elongate shaft portion of the device for the installation and securement of multiple ring electrodes on the shaft.

### BACKGROUND

Elongate catheter-based cardiovascular medical devices, such as electrophysiology (EP) catheters, can be used in a variety of diagnostic and/or therapeutic procedures to diagnose and/or correct medical conditions such as atrial arrhythmias, including for example, ectopic atrial tachycardia, atrial fibrillation, and atrial flutter. Arrhythmias can produce a variety of medical conditions including irregular heart rates, loss of synchronous atrioventricular contractions, and stasis of blood flow in a chamber of a heart, which can lead to a variety of other symptomatic and asymptomatic ailments and even death. The catheters can include multiple ring-shaped electrodes (or simply ring electrodes or electrodes) fixedly coupled to an elongate shaft portion configured to achieve these diagnostic and/or therapeutic purposes. For example, some electrodes can be configured to transmit electrical signals from the heart anatomy for diagnostics (e.g., cardiac mapping), while other electrodes can be configured to impart resistive heating or irreversible electroporation for therapeutics.

Radiofrequency (RF) ablation therapy can be conventionally used to treat various medical conditions. For example, RF ablation therapy may be used to treat cardiac arrhythmias. It is believed that the primary cause of atrial arrhythmia is stray electrical signals within the left or right atrium of the heart. An ablation catheter can be used to impart ablative energy (e.g., radiofrequency energy, electroporation, cryoablation, lasers, chemicals, high-intensity focused ultrasound, etc.) to create a lesion in the abnormal cardiac tissue, such that any undesirable electrical pathways within the heart can be potently limited or prevented. Electroporation is a non-thermal ablation technique in which an electric field is applied to tissue to induce pore formation in cellular membranes. The electric field from electrode(s) can be applied in a pulse train of relatively short duration pulses that last, for example, from a nanosecond to several milliseconds. When electroporation is applied to tissue in an in vivo setting, the cells in the tissue are subjected to a trans-membrane potential to induce the pore formation in the cellular membranes. Electroporation may be reversible (i.e., the induced pores are temporarily formed) or irreversible (i.e., the induced pores remain open and induce cellular destruction). In the field of cardiovascular diseases, irreversible electroporation can be used to induce cell destruction in the abnormal cardiac tissues that may cause any undesirable electrical pathways within the heart, thereby achieving similar, and possibly superlative, therapeutics to conventional RF ablation.

As an elongate medical device configured to provide an access to the heart anatomy and conduct relevant medical procedures (e.g., RF ablation, irreversible electroporation, cardiac mapping, etc.), a cardiovascular catheter generally consists of multiple shaft sections, including a proximal shaft section, a deflectable shaft section, and a distal functional shaft section (or alternatively referred as electrode shaft section) disposed at, and adherently interconnected to, the distal end of the deflectable shaft section. The proximal shaft section of an elongate catheter is generally coupled with a handle and adherently interconnected with the deflectable shaft of the catheter. Typically, the deflectable shaft section of the catheter contains a pull ring disposed at its distal end and one or more pull wires coupled to the pull ring, wherein the pull wire(s) passes through the proximal shaft section and then coupled to an activating mechanism residing within the handle. Therefore, steering forces imposed at the handle can be effectively transmitted through the proximal shaft section to properly deflect or curve the deflectable shaft section in different orientations, such that the functional (or electrode) shaft section, including various related functional components (e.g., electrodes, sensors, etc.), can be desirably positioned within the heart anatomy for intended medical procedures. Examples of catheters with different shaft sections, in particular distal electrode shaft sections comprising ring electrodes, are disclosed in U.S. patent nos. 5,524,337, 5,855,552, and 6,032,061, and 7,914,515.
US 2013/150808 A1 describes a catheter sheath and a method of manufacturing. The catheter sheath comprises a tubular member connected to a catheter handle, the tubular sheath including one or more electrical conductors and one or more electrode rings attached to the tip of the tubular member. The tubular member further includes at least one opening that allows the one or more electrical conductor to be electrically connected to the one or more electrode rings. The electrode ring is slid over the tubular member so as to cover the opening. The tubular member and the electrode ring are subjected to heat treatment by induction heating so that the material of the tubular member melts locally around the electrode ring to form a seal between the tubular member and the electrode ring.

### BRIEF SUMMARY

The present disclosure relates to catheters used during medical procedures such as, for example, diagnostic and therapeutic procedures to detect and/or correct medical conditions such as atrial arrhythmias (e.g., ectopic atrial tachycardia, atrial fibrillation, and atrial flutter). In many embodiments, a catheter includes an elongate electrode shaft section comprising one or more rubber members for improved securement of one or more electrodes to the elongate electrode shaft section. When electrodes (e.g., ring electrodes) are radially reduced in dimensions (e.g., via mechanical swaging) to encircle the shaft, the rubber members of the electrode shaft section under compression, compared to other layers of the electrode shaft section made of a different material e.g., thermoplastic polymers, will exhibit some long-term, hyperelastic material strains with minimal compression sets of material to be able to impart some long-lasting, high push-back forces against the electrodes for effectively maintaining the interference fits between the electrodes and the shaft. For example, the electrode shaft section has a composite tubular structure comprising multiple layers with same or different material properties. The one or more electrodes can be secured in place due to push-back radial forces/pressure from layers of the electrode shaft section. However, the layers of the electrode shaft section will exhibit decaying or time-evolving loss of the push-back radial force against the electrodes due to viscoelastic behavior of polymer (e.g., permanent set, stress relaxation, etc.). To enhance securement of the electrodes, the catheter shaft assembly herein is provided with the one or more members made of a highly resilient rubber or thermosetting elastomer in addition to other layers made of same or different thermoplastic polymer(s), including thermoplastic elastomers. The rubber members advantageously imparts the electrode shaft section with the high material resiliency and hyperelasticity with minimal compression set, while the other thermoplastic layers provides good manufacturability for the incorporation of the rubber member(s) into the shaft via thermal fusion bonding or melt reflow. In some embodiments, the electrode shaft section onto which ring electrodes are mounted can be disposed at, and adherently interconnected to, the distal end of a deflectable shaft section, such that the electrode shaft section, along with the deflectable shaft section, can be articulated to navigate through a tortuous path through a patient's vasculature. Advantageously, one or more rubber members of the electrode shaft section can sustain higher material strains and stresses for securely holding the electrodes in place during operation.

Thus, in one aspect, a catheter shaft assembly is described. The catheter shaft assembly includes an elongate tubular inner layer, a first rubber member disposed on an exterior surface of the elongate tubular inner layer, an elongate outer layer that covers an exterior surface of the first rubber member or the elongate tubular inner layer, and one or more ring electrodes attached to and encircling the elongate outer layer or the first rubber member, wherein each of the ring electrodes induces a radial hyperelastic deformation of the first rubber member that inhibits loss of an interference fit between the ring electrode and the elongate outer layer or the first rubber member. In some embodiments, the first rubber member encircles and extends along a first length of the elongate tubular inner layer.

In many embodiments, the elongate tubular inner layer is made of a thermoplastic or thermoplastic elastomer material. The thermoplastic or thermoplastic elastomer material of the elongate tubular inner layer has a durometer of greater than Shore D40. For example, the thermoplastic or thermoplastic elastomer material of the elongate tubular inner layer has a durometer in the range of Shore D60 to D85. In many embodiments, the outer layer is made of a thermoplastic or thermoplastic elastomer material. In some embodiments, the thermoplastic or thermoplastic elastomer material of the outer layer is the same as that of the elongate tubular inner layer. In some embodiments, the thermoplastic or thermoplastic elastomer material of the outer layer is different from that of an elongate tubular inner layer and has a durometer of less than Shore D60. In many embodiments, the first rubber member has a durometer equal to or less than Shore A65. The first rubber member is formed of a silicone rubber compound. In some embodiments, the first rubber member is chemically treated to impart chemical compatibility with at least one of the elongate tubular inner layer and the elongate outer layer.

In many embodiments, the elongate tubular inner layer has the same length as the elongate outer layer. In many embodiments, the first rubber member is elongated, and has a length equal to or shorter than the elongate tubular inner layer or the elongate outer layer. The first rubber member can be disposed between the elongate tubular inner layer and the elongate outer layer.

In some embodiments, the catheter shaft assembly further includes a second rubber member. Each of the first rubber member and the second rubber member has a length equal to or longer than a ring electrode of the one or more ring electrodes. The first rubber member is longitudinally spaced apart from the second rubber member along the elongate tubular inner layer. The catheter shaft assembly further includes a third rubber member having a length equal to or longer than a ring electrode of the one or more ring electrodes. The first, second, and third rubber members are longitudinally spaced apart from each other at equal distances or varying distances.

In some embodiments, the catheter shaft assembly further includes a second rubber member and a third rubber member. The first rubber member, the second rubber member, and the third rubber member are circumferentially distributed and spaced apart along the length of the elongate tubular inner layer. Each of the one or more ring electrodes induces a radial hyperelastic deformation on each of the first rubber member, the second rubber member, and the third rubber member that inhibits loss of the interference fit between each of the ring electrodes and the elongate outer layer , the first rubber member, the second rubber member, or the third rubber member. In some embodiments, the catheter shaft assembly further includes a fourth rubber member, a fifth rubber member, and a sixth rubber member. The first rubber member, the second rubber member, the third rubber member, the fourth rubber member, the fifth rubber member, and the sixth rubber member are circumferentially distributed and spaced apart along the first length of the elongate tubular inner layer. Each of the one or more ring electrodes induces a radial hyperelastic deformation of each of the first rubber member, the second rubber member, the third rubber member, the fourth rubber member, the fifth rubber member, and the sixth rubber member that inhibits loss of the interference fit between the one or more ring electrodes and the elongate outer layer, the first rubber member, the second rubber member, the third rubber member, the fourth rubber member, the fifth rubber member, or the sixth rubber member.

In some embodiments, at least one of the first rubber member and the second rubber member comprises a protruding portion shaped to enhance coupling with the elongate outer layer. For example, the first rubber member comprises a protruding portion shaped to enhance coupling with the outer layer or the elongate tubular inner layer. In some embodiments, the first rubber member is a tubular rubber member comprising at least one longitudinally oriented through slot or holes for facilitating melt flow-induced space filling and thermal fusion bonding of the elongate tubular inner layer, the outer layer, and the first rubber member.

In many embodiments, the catheter shaft assembly further includes a first electrode wire electrically connected to the first ring electrode. For example, the first rubber member includes a first electrode wire hole through which the first electrode wire extends. In some embodiments, the first rubber member further includes a potting hole configured to accommodate injection of a potting adhesive through the potting hole into a lumen defined by the elongate tubular inner layer.

In many embodiments, the catheter shaft further includes a metallic braided layer that is longitudinally disposed over the exterior surface of the elongate tubular inner layer and under an interior surface of the first rubber member.

In another aspect, a method of manufacturing a catheter shaft is described. The method involves forming a tubular inner thermoplastic layer, forming a first shaft assembly by placing or optionally in situ forming a first rubber member on an exterior surface of the tubular inner thermoplastic layer, forming a second shaft assembly by placing an outer thermoplastic layer over the first shaft assembly, forming a bonded catheter shaft by thermal-fusion bonding between the inner and outer thermoplastic layers to affix the first rubber member in position within the second shaft assembly; and attaching a first ring electrode to the bonded catheter shaft so that the first ring electrode induces a radial hyperelastic deformation of the first rubber member that inhibits loss of an interference fit between the first ring electrode and the bonded catheter shaft. In many embodiments, the first rubber member encircles and extends along a first length of the bonded catheter shaft. The first rubber member has a durometer equal to or less than Shore A65. The first rubber member is made of a silicone rubber compound.

In many embodiments, forming the first shaft assembly comprises placing or optionally in situ forming a plurality of discrete rubber members on the exterior surface of the tubular inner thermoplastic layer so that the discrete rubber members are circumferentially distributed and spaced apart along a first length of the tubular inner thermoplastic layer, wherein the plurality of discrete rubber members comprises the first rubber member. In some embodiments, one or more of the discrete rubber members include a protruding portion shaped to enhance coupling of the discrete rubber member with the outer thermoplastic layer.

In some embodiments, forming the first shaft assembly involves applying an adhesive to (e.g., temporarily affix the plurality of the pre-formed discrete rubber members onto) the exterior surface of the inner thermoplastic layer. The adhesive, when cured, is thermoplastic in nature. The adhesive includes a cyanoacrylate adhesive comprising at least one of: a cyanoacrylate monomer or oligomer.

In many embodiments, the thermal-fusion bonding comprises melting and reflowing the tubular inner thermoplastic layer and the outer thermoplastic layer of the second shaft assembly under radial compressive pressure. Forming the bonded catheter shaft further includes placing a heat-shrinkable tube over the outer thermoplastic layer prior to heating the second shaft assembly. In some embodiments, the method further involves pre-treating the first rubber member with one or more silane coupling agent to impart chemical compatibility with at least one of the inner thermoplastic layer or the outer thermoplastic layer e.g., to enhance melt adhesion of the inner and outer thermoplastic layers with the first rubber member.

In many embodiments, attaching a first ring electrode to the bonded catheter shaft comprises swagging the first ring electrode onto the bonded catheter shaft using a swaging die.

### BRIEF DESCRIPTION OF THE DRAWINGS

The accompanying drawings, which are incorporated in and constitute a part of the specification, illustrate one or more embodiments and, together with the description, explain these embodiments. The accompanying drawings have not necessarily been drawn to scale. Any values dimensions illustrated in the accompanying graphs and figures are for illustration purposes only and can or cannot represent actual or preferred values or dimensions. Where applicable, some or all features cannot be illustrated to assist in the description of underlying features.
FIG. 1 is a catheter, in accordance with some embodiments of the present disclosure.
FIG. 2(a) is an example catheter shaft portion including example rubber member for electrode shaft section of the catheter of FIG. 1 in a longitudinally exploded view.
FIG. 2(b) shows a cross-section view of the electrode shaft section of FIG. 2(a).
FIG. 2(c) is an enlarged portion of the cross-section of FIG. 2(b)
FIG. 2(d) illustrates example forces acting on an electrode of the electrode shaft section.
FIG. 2(e) illustrates example forces acting on electrode shaft section layers underneath the electrode of FIG. 2 (d).
FIG. 2(f) illustrates a rubber member with slots.
FIG. 2(g) illustrates a rubber member with holes.
FIG. 3 is another example catheter shaft portion including another example rubber member for an electrode shaft section of the catheter of FIG. 1 in a longitudinally exploded view and in a cross-section view.
FIG. 4 is another example catheter shaft portion including another example rubber member for an electrode shaft section of the catheter of FIG. 1 in a longitudinally exploded view and in a cross-section view.
FIG. 5 is an electrode shaft section showing electrodes coupled to the shaft of the catheter of FIG. 1.
FIG. 6 is a longitudinal-section view of the electrode shaft section showing electrodes coupled to the shaft of the catheter of FIG. 5.
FIG. 7 is an enlarged view of the longitudinal-section view of FIG. 6 showing different layers of the electrode shaft section including a rubber member.
FIG. 8 illustrates different shaft sections of another catheter similar to the catheter shown in FIG. 1.
FIG. 9 illustrates an electrode shaft section of the catheter of Figure 8 without a rubber member.
FIG. 10 is a graph illustrating relaxation behavior of some compressive material stress over time for different polymer materials under a given compression.
FIG. 11 is method of manufacturing the electrode shaft section of the catheter of FIG. 1 including a rubber member.
FIG. 12 is another example method of manufacturing an intimately bonded distal shaft assembly comprising an integral electrode shaft section of the catheter of FIG. 1 including a rubber member.
FIG. 13 is a schematic and block diagram of an electrophysiology catheter system for RF ablation or electroporation therapy that can include the catheter of FIG. 1.

### DETAILED DESCRIPTION OF THE DISCLOSURE

The description set forth below in connection with the appended drawings is intended as a description of various embodiments of the disclosed subject matter and is not necessarily intended to represent the only embodiment(s). In certain instances, the description includes specific details for the purpose of providing an understanding of the disclosed embodiment(s). However, it will be apparent to those skilled in the art that the disclosed embodiment(s) can be practiced without those specific details. In some instances, well-known structures and components can be shown in block diagram form in order to avoid obscuring the concepts of the disclosed subject matter.

The present disclosure provides a catheter suitable for use in the human vasculature for known medical procedures, such as cardiac ablation, cardiac mapping, irreversible electroporation, etc. For purposes of description and explaining the concepts, the present disclosure will be described in connection with ring electrodes that are mounted on a distal shaft section of the catheter e.g., via mechanical swaging. The distal shaft section with electrodes of the catheter herein comprises one or more highly resilient, rubber members, which will, upon instant compressive deformation imposed by the swaging of the electrodes, be able to sustain some counteractive, elastic push-back force against the electrode(s) to tightly secure the interference fit (e.g., pressed fit or friction fit) between the electrodes and the shaft for a long term. It is contemplated, however, that the described features may be incorporated into any number of catheters or introducers as would be appreciated by one of ordinary skill in the art.

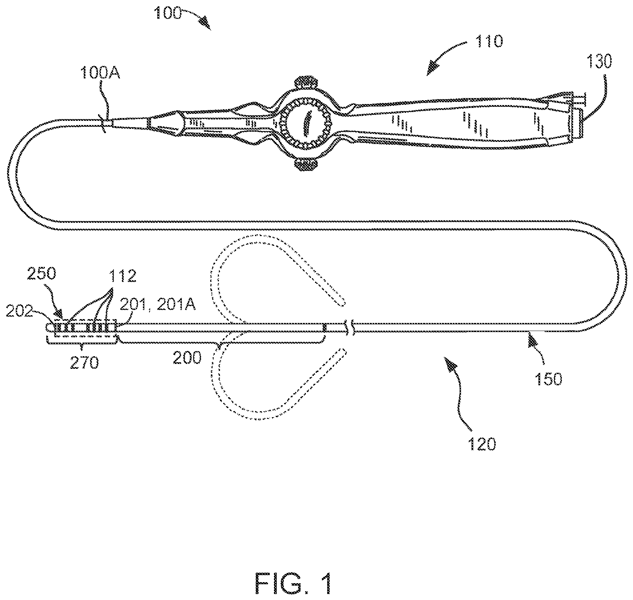

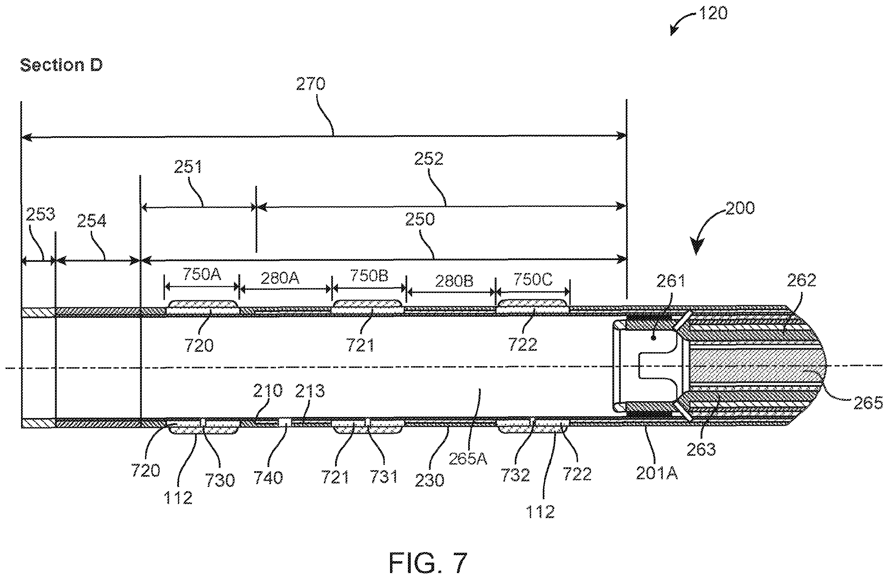

Referring now to the figures, in which like reference numerals refer to the same or similar features in the various views, FIG. 1 shows a catheter 100 with a handle 110, in accordance with many embodiments. The catheter 100 includes an elongate shaft 120 comprising a distal shaft section 250 onto which electrodes 112 are mounted e.g., via mechanical swaging. In many embodiments, the proximal end 201 of the distal shaft section 250 can be adherently attached to the distal end 201A of a deflectable shaft section 200 of the catheter 100. The distal shaft section 250 has a distal end 202 that may be adherently attached to other functional shaft sections (for example, for adopting an ablation tip & assembly and for accommodating sensors), such that the distal shaft section with electrodes, when integrated with the other functional shaft sections, may be alternatively identified as a distal functional shaft assembly (FSA) 270 of the catheter 100 (FIG. 7). A proximal portion 100A of the proximal shaft section 150 is coupled to a handle 110. The handle 110 is configured and operable to selectively curve the deflectable shaft section 200 together with the distal shaft section 250 with electrodes and other applicable functional shaft sections. For example, the deflectable shaft section 200, along with the distal shaft section 250, is configured to be selectively deflected in either of two directions as illustrated to accommodate navigation of the elongate shaft 120 through a patient's vasculature and/or positioning/orientation of the distal shaft section 250 of the catheter 100 within the heart anatomy during a medical procedure.

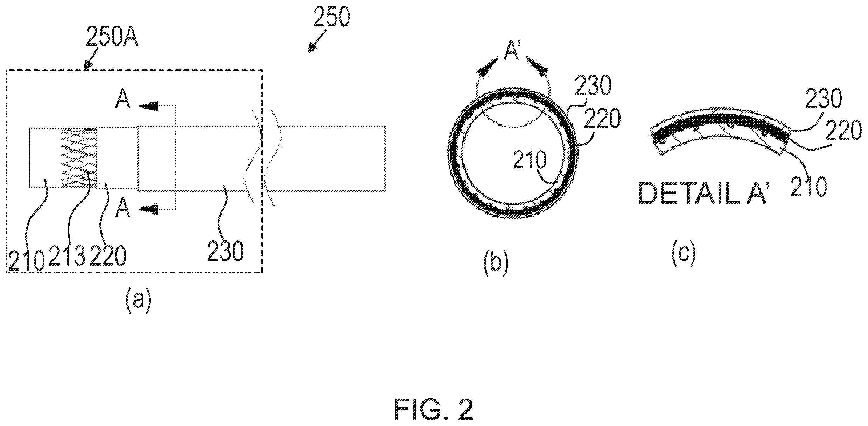

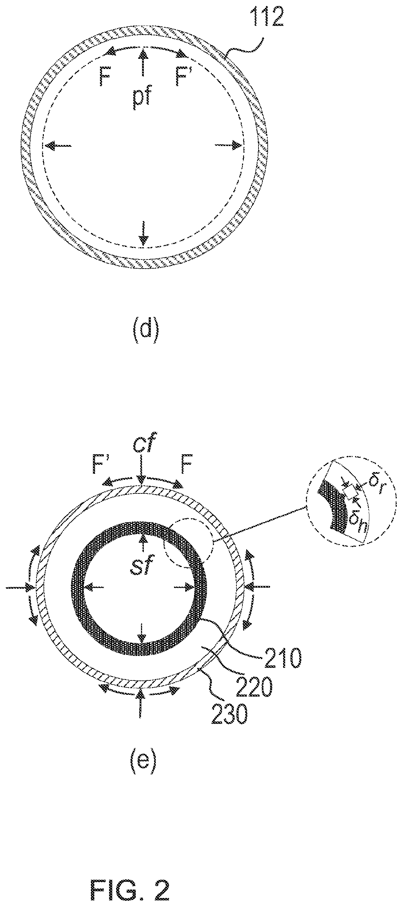

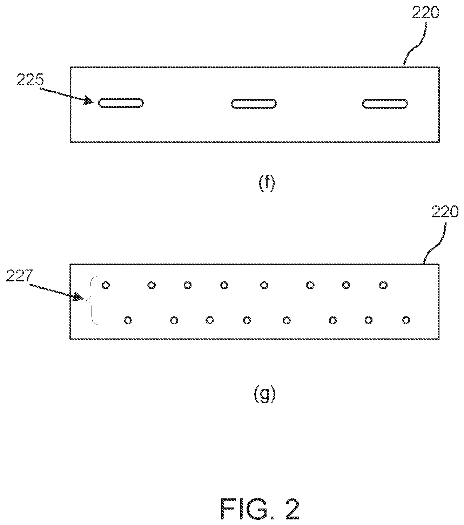

In many embodiments, the elongate shaft 120 has a composite, hollow shaft structure. The elongate shaft 120 includes various shaft sections with varying mechanical properties (e.g., stiffness, rigidity, flexibility, etc.), and/or may contain different electrical and functional components or assemblies, such as conductors or wires, magnetic sensors, optical force sensors, etc. The elongate shaft 120 can be made of same or different materials to collectively achieve a desired mechanical performance for a particular shaft section of the catheter 100. According to the present disclosure, the distal shaft section 250 or at least a portion of the distal shaft section 250, includes one or more rubber members configured to provide improved securement of electrodes 112. For example, the distal shaft section 250 comprises an elongate tubular shaft structure (e.g., see FIGS. 2-4), in which one or more rubber members or other hyperelastic member (e.g., 220, 320, 420) are disposed over an inner layer 210 or between an inner layer 210 (e.g., made of thermoplastic material) and an outer layer 230 (e.g., made of thermoplastic material). In many embodiments, one or more electrodes 112 are mounted to compressively encircle the distal shaft section 250 via mechanical swaging, so as to form the interference fit or pressed fit between the electrodes 112 and the distal shaft section 250. Swaging-induced hyperelastic compressive material strains within the distal shaft section 250 comprising the one or more rubber members can make the distal shaft section 250 effectively sustain a counteractive push-back force against the electrode(s) that inhibits the loss of an interference fit between the one or more ring electrodes and the distal shaft section 250. A hyperelastic material such as thermosetting elastomer or rubber is a material that respond elastically even when they are subjected to large deformations. The material shows both a nonlinear material behavior as well as large shape changes. Hyperelastic material can be characterized by large elastic deformations of order of 100 to 700% which are largely recoverable, i.e., the initial shape can be almost completely (e.g., more than 95% of the initial shape) recovered when load is removed. Example structure and configuration of the one or more rubber members or other hyperelastic material for the distal shaft section 250 are further discussed with respect to FIG. 2 through FIG. 4.

FIG. 2 illustrates an example construction of a portion 250A of a distal shaft section 250 with electrodes comprising at least one rubber member for the elongate shaft 120 of the catheter 100. The distal shaft section 250 or one or more portion (e.g., 250A) thereof includes one or more rubber members configured to provide improved securement of the electrodes 112 (e.g., ring electrodes) to the shaft of the distal shaft section 250. In the illustrated embodiment, the elongate distal shaft section 250 includes an elongate tubular inner layer made of thermoplastic polymer material, i.e., an inner layer 210, a tubular rubber member 220, and a tubular outer layer 230. The tubular rubber member 220 extends along a length equal to or slightly shorter than the length of the distal shaft section 250, while the inner and outer layers 210 and 230 extend the full length of distal shaft section. In some embodiments, the elongate distal shaft section 250 with electrodes comprises the tubular layers 210, 220, and 230 in same or different thicknesses and has its inner and outer thermoplastic layers made of the same or different thermoplastic polymer materials. Accordingly, the inner layer 210 and the outer layer 230 can be interchangeably referred as the inner thermoplastic layer 210 and the outer thermoplastic layer 230, respectively, without limiting the scope of the present disclosure.

In many embodiments, the distal shaft section 250 includes the inner thermoplastic layer 210 and the outer thermoplastic layer 230 that are chemically similar or compatible to each other but may have the same or different mechanical properties of polymer and where the tubular rubber member or layer 220 is adherently sandwiched between the thermoplastic layers 210 and 230. For example, the outer thermoplastic layer 230 can be made of a thermoplastic elastomer material having a durometer of less than Shore D40 (e.g., poly(ether block amide) copolymers, poly(ether-co-ester) copolymers, thermoplastic polyurethanes, thermoplastic olefins, olefinic thermoplastic vulcanizates, and the like), while the inner thermoplastic layer 210 can be made of a thermoplastic elastomer or thermoplastic having a durometer of greater than Shore D50, preferably greater than Shore 70D (e.g., nylons, polyesters, poly(bisphenol A carbonate), polyolefins, polysulfone, etc.). The tubular rubber member 220 can be made of a highly resilient rubber material having a durometer from Shore A40 to Shore A90 and good thermal stability of material, e.g., silicone rubber. The electrodes 112 can be made of 90Pt: 10Ir.

In some embodiments, each of the inner thermoplastic layer 210, the tubular rubber member 220, and the outer thermoplastic layer 230 may have same longitudinal lengths. In some embodiments, the inner thermoplastic layer 210, the tubular rubber member 220, and the outer thermoplastic layer 230 may have different longitudinal lengths. For example, the inner thermoplastic layer 210 and the outer thermoplastic layer 230 may have same longitudinal lengths and are axially extended to the full length of the distal shaft section 250 (e.g., a length between the proximal end 201 and the distal end 202). The tubular rubber member 220 may have a shorter longitudinal length than the thermoplastic layers 210 and 230, such that two thermoplastic layers 210 and 230 are integrally adhered together where the tubular rubber member or layer 220 is absent at and near two opposite ends 201 and 202 of the distal shaft section 250.

In some embodiments, there may be more than one tubular rubber members 220 longitudinally disposed along the distal shaft section 250, each of which has a length equal to, or slightly longer than, the length of the corresponding ring electrodes, such that the individual tubular rubber members 220 can be longitudinally disposed and fully encapsulated by the thermoplastic layers 210 and 230 having an elongate tubular structure to form different shaft portions where electrodes are to be mounted, while the thermoplastic layers 210 and 230 can be intimately adhered to each other where the tubular rubber members 220 or electrodes 112 are absent.

In many embodiments, the inner thermoplastic layer 210 extends longitudinally e.g., between the proximal end 201 and the distal end 202 of the elongate distal shaft section 250. The inner thermoplastic layer 210 may further extend longitudinally up to the entire deflectable shaft section 200, or even continuously extend to the proximal shaft section 150 (i.e., the entire length of an elongate shaft 120).

In many embodiments, the inner thermoplastic layer 210 includes a center lumen to accommodate various electrical components for catheter 100 (e.g., conductors to electrodes and leading conductive wires to sensors, etc.), and/or functional assemblies (e.g., assemblies associated with magnetic positioning sensor, optical force sensor, etc.), and/or other accessory components (e.g., pull wires, irrigation fluid tube, and etc.) as required to perform a medical procedure. Some functional assemblies, e.g. sensor assemblies, include shell-like sensor holders that are made of high-performance engineering polymer materials like polysulfone, polyetherimide, poly(ether ether ketone), etc.) may make direct contact with the interior surface of the inner thermoplastic layer 210, such that the inner thermoplastic layer 210 of the distal shaft section 250 is largely supported and strengthened to prevent any radial clasping when the electrodes 112 are forcibly swaged to impose radial compression force against the distal shaft section 250.

The tubular rubber member 220 is placed or optionally in situ formed over the inner thermoplastic layer 210 that may be further internally strengthened or supported (by relatively rigid shell-like sensor holders). The tubular rubber member 220 is flexible and tightly fits on an exterior surface of the inner thermoplastic layer 210. The outer thermoplastic layer 230 tightly fits on the exterior surface of the tubular rubber member 220. The tubular rubber member 220 can be adherently integrated with the thermoplastic layers 210 and 230 such that there is no relative movement between each other. For example, the relative disposition of the layers 210, 220, and 230 is shown in FIG. 2(b). FIG. 2(b) illustrates a cross-section view taken along a section line A-A in FIGS. 2(a), and 2(c) shows an enlarged portion A' of the cross-section. FIGS. 2(b) and 2(c) show an interior surface of the tubular rubber member 220 is intimately integrated with the exterior surface of the inner thermoplastic layer 210. Further, the exterior surface of the tubular rubber member 220 is intimately integrated with the interior surface of the outer thermoplastic layer 230. The integral thickness of the distal shaft section comprising the layers 210, 220, and 230 with the electrode 112 can be configured to maintain a desired outer diameter comparable to that of the deflectable shaft section 200 or the elongate shaft 120 to facilitate insertion and retraction through blood vessels within a patient. For example, FIGS. 2(b) and 2(c) illustrate relative thickness and diameters of the inner thermoplastic layer 210, the tubular rubber member 220, and the outer thermoplastic layer 230. In particular, an outer thermoplastic layer 230 can be very thin and made of a thermoplastic elastomer material that is considerably softer than the inner thermoplastic layer 210. Thus, advantageously, the rubber member 220 can largely retain its high hyperelastic material deformation to effectively provide push-back forces, via the thinner and softer thermoplastic outer layer 230, against an electrode 112, while the inner layer 210 would only bear a small material deformation to dissipate or become unrecoverable over time, thereby electrodes can be securely coupled against the push-back forces in the long term.

Unlike the inner and outer thermoplastic layers 210 and 230, the tubular rubber members 220 cannot be re-shaped or re-processed by heating. Hence, in many embodiments, the tubular rubber members 220 as shown in FIG. 2 have a particular structural feature of e.g., at least one elongate slots 225 (see FIG. 2(f)) or multiple holes 227 (see FIG. 2(g)) through the wall of the rubber members 220. The slots 225 and holes 227 may be staggered formation, evenly distributed, or other geometric formation without limiting the scope of the present disclosure. When subjected to heating, the inner and outer thermoplastic layers 210 and 230 can reflow through the slots or holes and be reshaped to encapsulate the rubber members 220 intimately and adherently within the distal shaft section 250 as an integral entity.

In many embodiments, the tubular rubber member 220 extend longitudinally e.g., between the proximal end 201 and the distal end 202, or a portion therebetween. For example, the tubular rubber member 220 can have a longitudinal length defined by a distance between end electrodes 112 e.g., a distal electrode and a proximal electrode. The tubular rubber member 220 may be shorter in length than the inner thermoplastic layer 210 and/or the outer thermoplastic layer 230.

In some embodiments, the tubular rubber member 220 has a continuous length extending along the distal shaft section 250. In some embodiments, multiple pieces of the tubular rubber member 220 may be longitudinally spaced from each other. For example, the distal shaft section 250 may include one or more tubular rubber members 220, each rubber member disposed at a longitudinal location along the distal shaft section 250 that corresponds to a particular electrode of the electrodes 112 (e.g., as shown in FIG. 7). For example, a first tubular member may be disposed at a first longitudinal location of the distal shaft section 250 where a first electrode of the electrodes 112 is mounted, a second tubular member may be disposed at a second longitudinal location of the distal shaft section 250 where a second electrode of the electrodes 112 is mounted, etc. Each of these tubular rubber members 220 may have a longitudinal length comparable to or slightly longer than a corresponding electrode to be mounted along the distal shaft section 250 e.g., by mechanical swaging.

The tubular rubber member 220 can largely sustain the swaging-induced hyperelastic material strains and associated compressive material stresses for a considerably longer duration of time than other thermoplastic layers of the distal shaft section 250. The tubular rubber member 220 facilitates improved securement of the electrode 112 by providing the long-lasting push-back forces against the electrode 112 in response to the hyperelastic material strains and stresses within the shaft portion 250A, at which the electrode 112, when mounted, imposes constant compressive deformation on the shaft 250A. The tubular rubber element 220 has a short relaxation time of material and can swiftly attain its steady state of stress relaxation with minimal permanent set (e.g., minimal unrecoverable or viscous or inelastic material strains), such that the shaft portion 250A at the steady state can still counteractively impart a hyperelastic push-back force against the electrode 112 to maintain an interference fit between the electrodes 112 and the distal shaft section 250.

Mechanical swaging is a forging or cold-working process, during which the diameters of an electrode 112 are forcibly, "permanently" reduced by applying a rotary die, thus resulting in some compressive material strains in the hoop (or circumferential) direction ("*h*") and in the radial direction ("*r*") and the associated compressive material stresses (σᵣ and σₕ) within various constituent components (i.e., the layers 210, 220, and 230) of the shaft portion 250A. FIGS. 2(d) and 2(e) show the schematic, free-body force diagrams for the electrode 112 and the shaft portion 250A of the distal shaft section 250 comprising the rubber member 220, the inner thermoplastic layer 210, and a skin-like, flexible outer thermoplastic layer 230 over the exterior surface of the rubber member 220.

At and after mechanical swaging, the swaging-mounted electrode 112 will impose a radial compressive force (cf) that circumferentially distributes on the exterior surface of the shaft portion 250A, which will relax or decay with time because some compressive material strains are inelastic (i.e., unrecoverable) in nature or the associated compressive material stresses (i.e., σₕ and σᵣ) within the continuum comprised of various constituent layers of the shaft portion 250A will relax towards steady strained/stressed states. In many embodiments, the inner thermoplastic layer 210 is made of a rigid thermoplastic polymer or thermoplastic elastomer having a considerably higher material hardness than the rubber member 220 made of an elastomeric thermosetting or crosslinked polymer material and also the outer thermoplastic layer 230.

Hence, when a radial compressive force (cf) applied on the shaft portion 250A, the rubber member 220, as compared to other constituent thermoplastic layers 210 and 230 within the continuum of the shaft portion 250A, will qualitatively experience a much higher material strain that is hyperelastic in nature because of the highly resilient, elastomeric mechanical properties of the rubber material, including a small compression set (< 5% to 30%) and a significantly short characteristic time (τ) of stress relaxation of material. In contrast, the swaging-induced compressive material strains within the inner thermoplastic layer 210 of the shaft portion 250A are relatively small in magnitude and viscoelastic (or more viscous or plastic) in nature. Therefore, the swaging-induced material strains within the layer 210 tend to become unrecoverable and eventually take a high compression set (e.g., >50% to nearly 100%), and the associated material stresses within the layer 210 will decay with time. In contrast, the swaging-induced, relatively large hyperelastic material strains tend to recover and will not ever decay to none, and neither will the associated material stresses in relaxation towards the steady strained/stressed state of material, within the rubber member 220, thanks to low compression set and fast stress relaxation characteristics of the rubber material (e.g., <5 to 30%), such that the rubber member(s) 220 with the shaft portion 250A can counteractively impose a sustainable push-back force (pf) against the electrode(s) 112 at any portions of the shaft portion 250A where electrode(s) is mounted.

As shown in FIG. 2d, the shaft portion 250A, as a free body subjected to the only radial compressive force (cf) imposed by the electrode(s) 112, can be in force balance on its own, provided that the inner tubular thermoplastic layer 210 is mechanically strong to support and contain the rubber member 220 within the distal shaft section 250 (e.g., without any material failure or structural collapsing). Alternatively, the distal shaft section 250 may experience some additional radial force (sf) externally imposed by any components/assemblies that reside within the center lumen of the distal shaft section 250 (e.g., sensor holders). This radial force (cf) may advantageously provide a mechanical support to the distal shaft section 250 to prevent any possible structural collapsing when the shaft portion 250A is externally subjected to any swaging-induced radial compressive force (cf). Again, the shaft portion 250A, as a free body subjected to both radial compressive forces in opposite directions (cf and sf), are in force balance on its own. Following Newton's third law, the push-back force (pf) (against electrodes 112) is equal to the "relaxing" compressive force (cf) (against the shaft portion 250A) in magnitude but opposite in direction. The electrode 112 has substantially higher rigidity than the polymer materials of the shaft portion 250A. As such, the electrode 112, as a free body, is externally subjected to only "relaxing" push-back force (pf) that is circumferentially distributed on the interior surface of the electrode 112, can be in balance on its own without causing material creeping or expansion. Under the "relaxing" but highly sustainable radial compressive force (pf), there is a highly sustainable static frictional force (F or F') against the electrode(s) 112. That is, whenever the electrode 112 tends to become loose with respect to the shaft portion 250A in any directions (e.g., clockwise or counterclockwise, etc.), a counteracting static frictional force (F or F') in an opposite direction will spontaneously appear to secure the interference fit, which is also commonly known as friction fit, for inhibiting any movement of the electrode 112 with respect to the shaft portion 250A or the distal shaft section 250 of the catheter 100.

Additionally or alternatively, a braided layer 213 woven of metal material or other rigid material to provide structural rigidity and resilience. The braided layer 213 can be alternatively referred as metallic braided layer 213, without limiting the scope of the present disclosure. For example, the braided layer 213 may be multiple stainless-steel wires woven in some pattern that partly extend along the distal shaft section 250 from the deflectable shaft section 200 to provide additional structural rigidity and resiliency for the distal shaft section 250 (in FIG. 1). The metallic braided layer 213 may be disposed underneath the tubular rubber member 220 or between the inner thermoplastic layer 210 and the tubular rubber member 220.

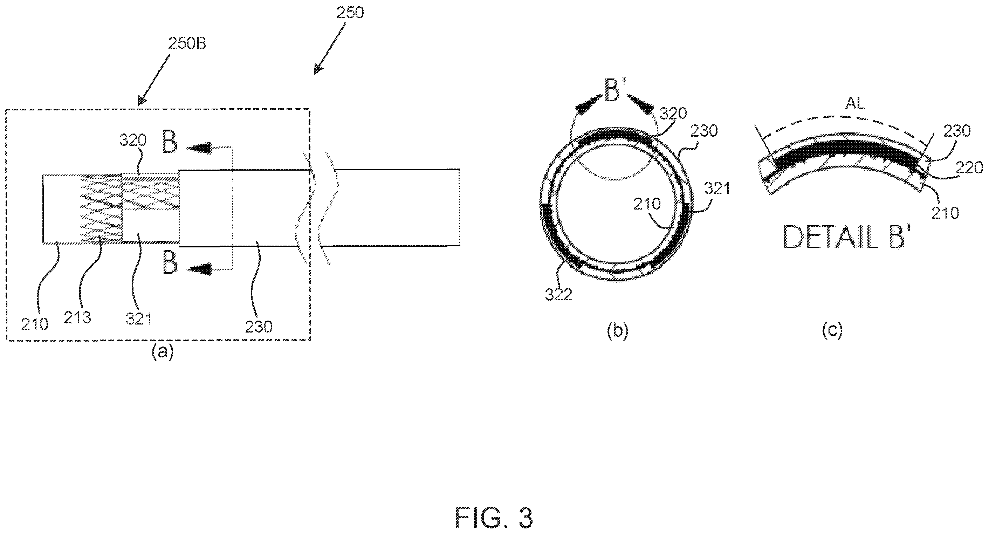

FIG. 3 illustrates another example construction of the distal shaft section 250, in particular a shaft portion 250B. The shaft portion 250B has one or more than one rubber members that are non-tubular (e.g., having an arc-shape) unlike a tubular rubber member of the shaft portion 250A in FIG. 2). Other layers e.g., 210 and 230 can be same as layers of the shaft portion 250A. In the illustrated embodiment, in FIG. 3, the shaft portion 250B of the distal shaft section 250 can include one or more longitudinally extending discrete rubber members (e.g., 320, 321, 322). The discrete rubber members (e.g., 320, 321, 322) can advantageously ensure secured attachment of the electrodes 112 in the same manner as the tubular rubber member 220 (in FIG. 2). For example, the discrete rubber members 320-322 provides the push-back forces in a similar manner as discussed with respect to FIG. 2 above. Example structure and distribution of the discrete rubber members can be seen in a cross-section view B-B in FIG. 3(b) and an enlarged view of a portion B' in FIG. 3(c).

In the illustrated embodiment, in FIG. 3(b), the distal shaft section 250 includes the inner thermoplastic layer 210, a group or set of discrete rubber members 320, 321, and 322 (for example, used in place of the tubular rubber member 220 in FIG. 2), and the outer thermoplastic layer 230. Additionally or alternatively, the metallic braided layer 213 may be included in the composite shaft structure of a distal shaft section 250. The group of the discrete rubber members 320, 321, and 322 may be longitudinally disposed at individual location corresponding to each ring electrodes along a length of the distal shaft section 250 or longitudinally extended for a length equal to or slightly less than the distal shaft section 250. The discrete rubber members 320, 321, and 322 in a group are circumferentially spaced apart from each other. For example, a first rubber member 320 is circumferentially spaced from a second rubber member 321 and a third rubber member 322. In many embodiments, the rubber members 320, 321, and 322 are evenly spaced over an exterior surface of the inner thermoplastic layer 210. An arcuate arc length (AL) (see FIG. 3(c)) of each of the discrete rubber members 320-322 can be the same so that equal amount of the push-back forces can be applied to the electrode 112 at different angular positions corresponding to the discrete rubber members 320-322. The push-back forces from the rubber members 320-322 disposed at locations corresponding to the electrodes will be similar to that shown in FIG. 2(b). However, along a circumferential arc-length portion between the discrete rubber members 320-322, the distal shaft section 250 comprises the inner and outer thermoplastic layers 210, 230, which will experience a slow relaxation of the push-back forces over time. These push-back forces from the layers may tend to eventually disappear because swaging-induced compressive material strains may become as permanent sets. Advantageously, the discrete rubber members 320-322 will be able to sustain the swaging-induced material strains and associated material stresses, thus imposing the desired push-back forces for the long-term securement of the electrodes 112 against the distal shaft section 250 or its portion 250B comprising the highly resilient rubber members.

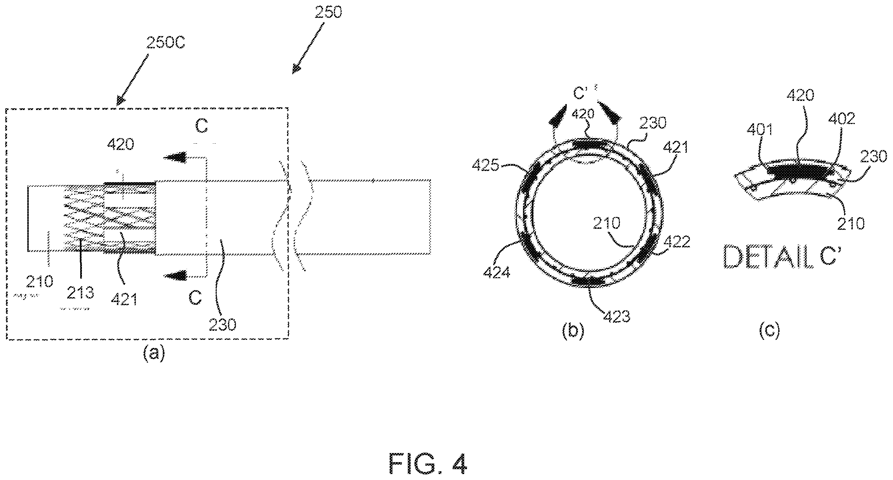

FIG. 4 illustrates an example construction of the distal shaft section 250, in particular a shaft portion 250C. The shaft portion 250C has one or more than one rubber members that are non-tubular (e.g., having an arc-shape) unlike a tubular rubber member of the shaft portion 250A in FIG. 2). The electrode shaft section 250 comprising the shaft portion 250C in FIG. 4 has similar construction as the distal shaft section 250 comprising the shaft portion 250B of FIG. 3, except that there are a group of six, instead of three, discrete rubber members in place of a tubular rubber member 220 for a shaft portion 250A of FIG. 2. It can be understood that the present disclosure is not limited by a number of discrete rubber members in a group and any appropriate number (e.g., 2, 3, 4, 5, 6, or more) of the rubber members for the group may be employed. Again, the discrete rubber members 420-425 in FIG. 4 can advantageously ensure secured attachment of the electrodes 112 in the same manner as the discrete rubber members 320-322 in FIG. 3. For example, the discrete rubber members 420-425 provide the sustainable long-term push-back forces in a similar manner as discussed with respect to FIG. 3 above. Example structure and distribution of the discrete rubber members 420-425 can be seen in a cross-section view C-C in FIG. 4(b) and an enlarged view of a portion C' in FIG. 4(c).

In the illustrated embodiment, in FIG. 4(c), a discrete rubber member (e.g., 420) has protruding portions (or side profiles) 401 and 402. The protruding portions 401 and 402 may be wing-like features formed at two opposite ends of the discrete rubber member 420. The protruding portions 401 and 402 create spaces to be filled by the polymer material of the thermoplastic layers 230 and/or 210. For example, the outer thermoplastic layer 230 can melt and spread around the protruding portions 401 and 402 to securely integrate the rubber member 420 to the outer thermoplastic layer 230.

In the illustrated embodiment, in FIG. 4(b), the elongate distal shaft section 250 includes the inner thermoplastic layer 210, six discrete rubber members 420-425, and the outer thermoplastic layer 230. Additionally or alternatively, the metallic braided layer 213 may be included in the distal shaft section 250 comprising a shaft portion 250C. The group of the six discrete rubber members 420-425 extends along a length equal to or slightly longer than the length of a corresponding electrode 112 and are circumferentially spaced apart from each other. In many embodiments, the rubber members 420-425 are evenly spaced over an exterior surface of the inner thermoplastic layer 210. Each of the discrete rubber members 420-425 has an arc shape. An arcuate length of each of the discrete rubber members 420-425 can be same so that equal amount of push-back forces can be applied to the electrode 112 at different angular positions corresponding to the discrete rubber members 420-425. In some embodiments, such a shaft portion 250C longitudinally extend to a length equal to or slightly shorter than the distal shaft section 250. In some embodiments, there may be multiple shaft portions 250C longitudinally disposed along the length of the distal shaft section 250, while other shaft portions between individual shaft portions 250C may include the inner thermoplastic layer 210 and the outer thermoplastic layer 230 without any rubber members 420 to 425 (see FIG. 7) disposed therebetween.

It can be understood the present disclosure illustrates three and six discrete rubber members configuration in FIG. 3 and FIG. 4. respectively, by way of examples. The present disclosure is not limited a number of rubber members. The elongate distal shaft section 250 can include 2, 3, 4, 5, 6, or more discrete rubber members that extend longitudinally. Also, the longitudinal length of each of the discrete rubber members can be sufficient to continuously extend underneath all electrodes 112 or may further extend to be equal to the distal shaft section 250.

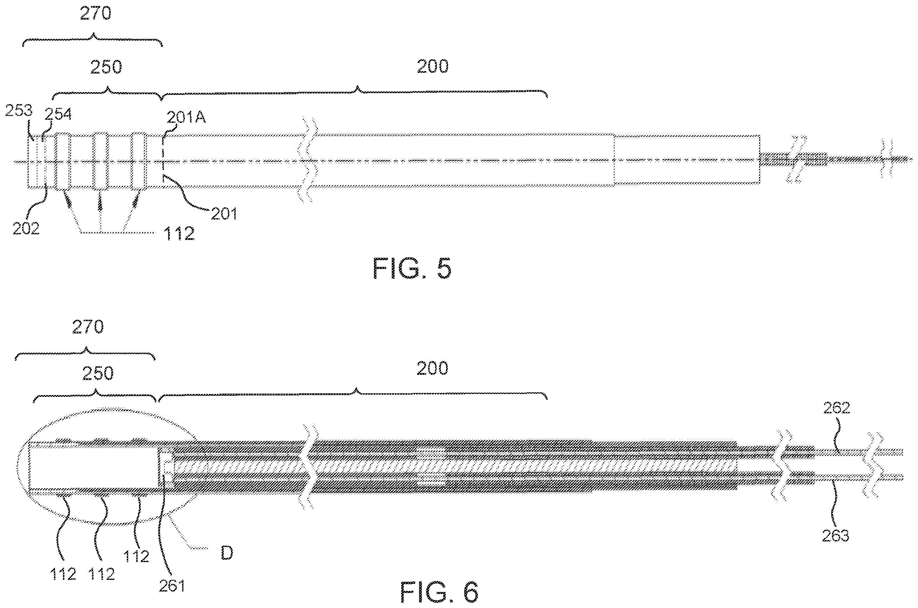

In many embodiments, referring to FIG. 5 through FIG. 7, another example configuration of one or more rubber members disposed in the distal shaft section 250 of catheter shaft 120 is discussed. FIG. 5 illustrates an assembled view and FIG. 6 illustrates a longitudinal-section view of the elongated shaft assembly 120 including a distal shaft section 250 onto which the electrodes 112 are mounted. The deflectable shaft section 200 is configured for steering the elongate shaft 120 through tortuous vasculature to deliver the distal shaft section 250 at a desired location within the heart anatomy of a patient.

In some embodiments, the distal shaft section 250 may be manufactured together with other functional shaft sections (e.g., a shaft section 253 for adopting an ablation tip & assembly and another shaft section 254 for accommodating and encircling sensor(s), etc.), thus forming a distal FSA 270 comprising the distal shaft section 250 on which electrodes are mounted. The distal FSA 270 is coupled to the deflectable shaft section 200, which is in turn coupled to the proximal shaft section 150, thus forming an elongate catheter shaft 120. In some embodiments, one or more layers e.g., the inner thermoplastic layer 210 and/or the metallic braided layer 213 may be continuously formed of the deflectable shaft section 200.

FIG. 7 illustrates an enlarged view of cross-section portion D (shown in FIG. 6) of the elongated shaft assembly 120 showing detailed structure and components in the distal shaft section 250 and the distal portion 201A of the deflectable shaft section 200. To impart various essential diagnostic and/or therapeutic functionality of an EP catheter, other shaft sections, such as a tip-fitting shaft section 253, a moisture-barrier shaft section 254, may be disposed at the distal front of the distal shaft section 250. The tip-fitting shaft section 253 is configured to distally receive a tip assembly (e.g., an assembly containing an ablation tip) used for a medical procedure, e.g., RF ablation, irreversible electroporation, etc. The moisture-barrier shaft section 254 is disposed between the tip-fitting shaft section 253 and the distal shaft section 250 to prevent moisture from entering the lumen of the moisture-barrier shaft section 254 where an optical sensor resides. The distal shaft section 250 is disposed between the moisture-barrier shaft section 254 and the deflectable shaft section 200. In many embodiments, a distal shaft section 250 may be seamlessly integrated, distally with a moisture-barrier shaft section 254, and proximately with a deflectable shaft section 200, as an integral entity via thermal fusion bonding or melt reflow.

In the illustrated embodiments, the distal shaft section 250 includes one or more shaft portions 750A, 750B or 750C with one or more rubber members (e.g., tubular rubber member of FIG. 2 and/or a group of discrete rubber members of FIGS. 3 or 4). Additionally, features may also be included to facilitate electrical connections and/or adhesion. For example, the distal shaft section 250 comprising the shaft portion 750A, 750B and 750C includes a plurality of discrete tubular rubber members (e.g., 720-722) axially or longitudinally spaced from each other. An individual tubular rubber member or an individual group of the discrete tubular rubber members (e.g., 720-722) encircles and extends along a respective length of the ring electrodes 112. For example, a first tubular rubber member 720 or a first group of discrete rubber members 720 extends underneath a first ring electrode 112, a second tubular rubber member 721 or a second group of discrete tubular rubber members 721 extends underneath a second ring electrode 112, and a third tubular rubber member 722 or a third group of discrete rubber members 722 extends underneath a third ring electrode 112, etc. The ring electrodes 112, when mounted on the distal shaft section 250, will reduce the outer diameter of the distal shaft section 250 to cause some hyperelastic material strains and the associated compressive material stresses within the rubber member(s) of the distal shaft section 250 comprising the shaft portions 750A, 750B, and/or 750C.

Other elongational shaft portions without any rubber members (e.g., 271 and 272) may be largely absent of any swaging-induced material strains & stresses and can retain a constant outer diameter. As such, the outer diameter of the distal shaft section 250 may vary from a shaft portion to another. In many embodiments, the outer shaft diameter for a shaft portion 750A, 750B and/or 750C that comprises one or more rubber members 220 is equal to, or slightly larger than, other shaft portion such as portion 280 that does not contain any rubber members 220, such that after the electrode(s) is mounted by mechanical swaging, there is a smooth transition in the shaft diameters Between different shaft portions along the distal shaft section 250.

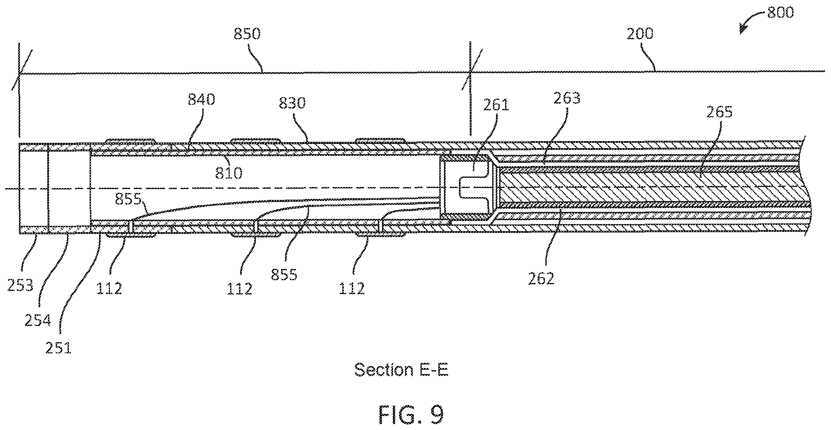

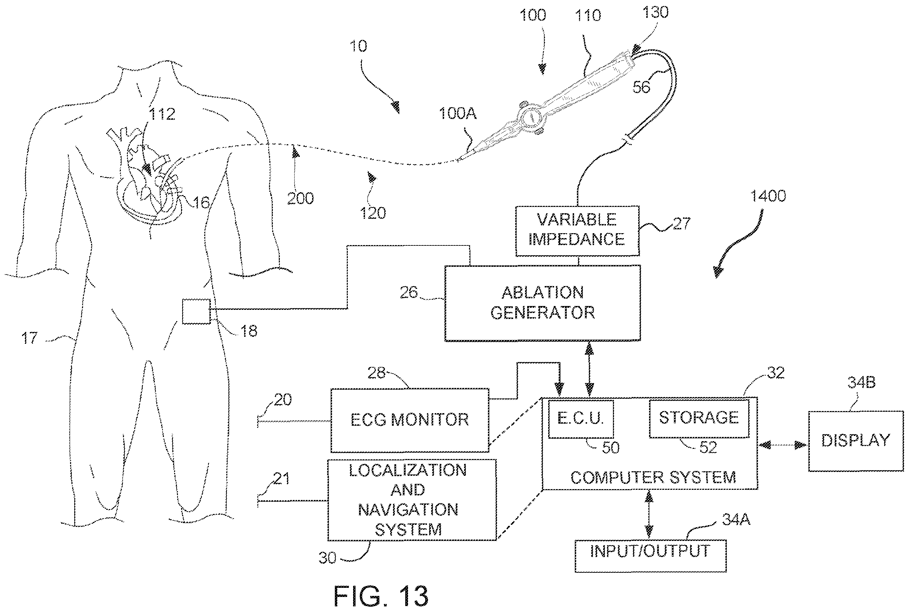

In some embodiments, at least one of the longitudinally-space rubber member 720-722 includes an electrode-wire hole, e.g., 730, 731, and 732, configured to accommodate routing of an electrode-wire (e.g., similar to wires 855 in FIG.9) coupled with a respective ring electrode of the one or more ring electrodes 112. The electrode-wire hole 730, 731, and 732 can be a through hole extending from an inner surface of the inner thermoplastic layer 210 to an exterior surface of the outer layer 230 or the rubber member 220. For example, the discrete tubular rubber members 720-722 include electrode wire hole 730-732, respectively, to electrically couple an electrode wire and route the wire through a lumen 265 of the elongate shaft 120 and the handle 110 where the wire is integrated with an electrode harness or connector 130 (FIG. 1). For example, an electrode-wire (e.g., wire 855 in FIG. 9) may be coupled with an electrode 112 by welding and strung through the center lumen 265 of the elongate shaft 120 until the handle 110 (see FIG. 1 and FIG. 13), and then coupled with an electrical connector 130 leading to a catheter system (e.g., see FIG. 13)

In some embodiments, distal shaft section 250 includes at least one adhesive potting hole 740 configured to accommodate the injection of a potting adhesive through the potting hole 740. In some embodiments, the potting hole 740 may be located in a portion (e.g., 280A or 280B) of the distal shaft section 250 between adjacent electrodes 112 where no rubber member 220 is present. For example, an adhesive potting hole 740 is located between the first tubular rubber member (or the first group of discrete rubber members) 720 and the second tubular rubber member (or the second group of discrete rubber members) 721. The potting hole 740 provides an access from the exterior surface of the distal shaft section 250 to an internal center lumen 265A of the distal shaft section 250. Through the potting hole 740, a potting adhesive can be injected into any spaces unoccupied by components within the lumen 265A of the distal shaft section 250 and in situ cured. The cured adhesive fixes various components (e.g., electrode-wires, a tip assembly component, sensors, etc.) disposed within the lumen 265A of the distal shaft section 250 in position to result in a solid, relatively rigid core that can mechanically strengthen the distal shaft section 250 for preventing the distal shaft section 250 from collapsing when the distal shaft section 250 is subjected to a radial compressive deformation or force (cf) upon mechanically swaging one or more electrode(s) 112. In some embodiments, a rigid core of the inner layer 210 can span approximately from a proximal end of the moisture-barrier tubular section 254 (where the optical force sensor resides) to the proximal end 201 of the distal shaft section 250 (or the distal end of the deflectable shaft section 201A) where a pull ring 261 resides.

Additionally, the distal shaft section 250 can include a braided shaft portion 252 and an unbraided shaft portion 251. The braided shaft portion 252 includes a metallic braided layer 213 circumferentially disposed over the exterior surface of an inner thermoplastic layer 210, while the unbraided section 251 does not include a metallic braided layer. In the illustrated embodiment, the metallic braided layer 213 of the distal shaft section 250 may extend proximally through the deflectable shaft section 200 or even an elongate shaft 120 to provide a smooth, reinforced and intimate integration with the deflectable shaft section 200. The unbraided shaft portion 251 of the distal shaft section 250 extends distally to effect an intimate and smooth integration with the tubular moisture-barrier shaft section 254 and the tip-fitting shaft section 253.

As illustrated in FIGS. 6 and 7, the deflectable shaft section 200 of the elongate shaft 120 internally includes components used for deflecting the distal FSA 270 comprising a distal shaft section 250 and/or other functional shaft sections (such as a tip-fitting shaft section 253 and a moisture-barrier shaft section 254, etc.). For example, the pull ring 261 is internally disposed at the distal end of the deflectable shaft section 200 and coupled with a pair of pull wires 262 and 263. The pull wires 262 and 263 extend proximally through the deflectable shaft section 260, the proximal shaft section 150, and then coupled with a steering mechanism that resides within the handle 110 (see FIG. 1).

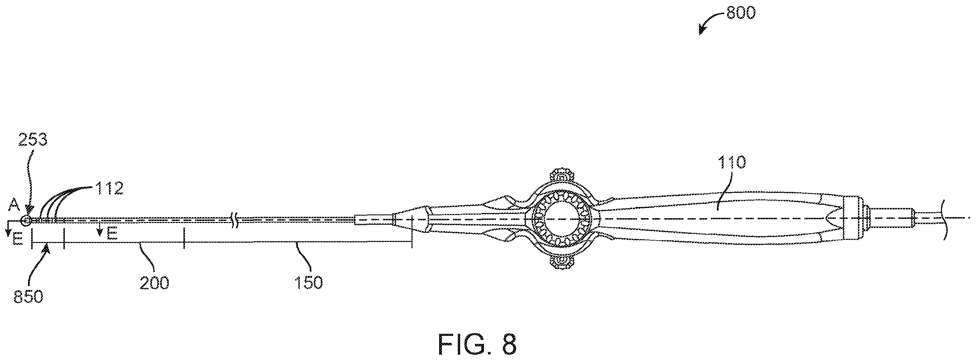

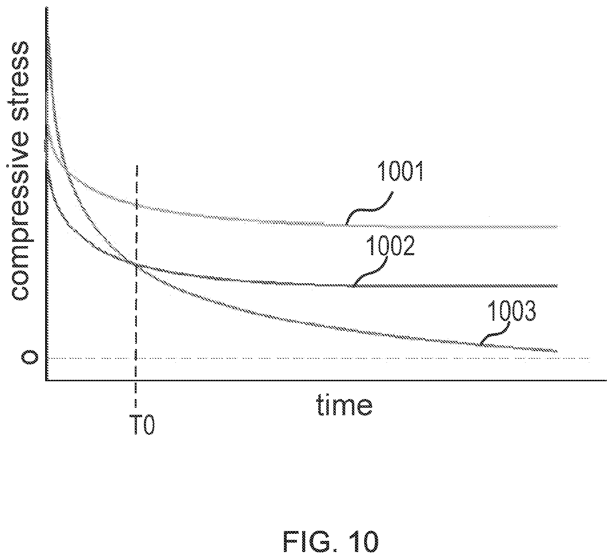

Referring to FIGS. 8 and 9, another catheter 800 including a distal shaft section 850 where electrodes 112 are mounted and the deflectable shaft section 200 is illustrated. The catheter 800 include several sections similar to the catheter 100 except for the distal shaft section 850. The distal shaft section 850, unlike the distal shaft section 250 comprising at least one shaft portion 750A, 750B and/or 750C. does not include any rubber member extending underneath the electrodes 112. As such, the distal shaft section 850 of the catheter 800 would have different mechanical responses to swaging-induced material strains and stresses compared to the distal shaft section 250 of the catheter 100. In the illustrated embodiment, the distal shaft section 850 includes an elongate tubular inner thermoplastic layer 810 (e.g., similar to the inner thermoplastic layer 210 of the catheter 100) and an elongate tubular outer thermoplastic layer 830 (e.g., similar to the outer thermoplastic layer 230 of the catheter 100) with an optional metallic braided layer 840 (e.g., similar to the braided layer 213 of the catheter 100). The electrode 112 are mounted onto the distal shaft section 850 comprised of the elongate tubular inner thermoplastic layer 810 and the outer thermoplastic layer 830 by swagging. However, the swaging-induced material strains within the thermoplastic layers 810 and 830 of the distal shaft section 850 are largely unrecoverable or inelastic in nature and tend to "permanently" take some compression sets of > 50% up to nearly 100%, because the distal shaft section 850 does not have one or more hyperelastic rubber members. As a result, the distal shaft section 850 cannot effectively retain the push-back force (pf) but may even decay to none, because of large loss of any recoverable material strains within the distal shaft section 850. While the catheter 100 including the distal shaft section 250 having the hyperelastic rubber members is highly resilient compared to the distal shaft section 850, as such advantageously the swaging-induced material strains within the rubber members 220 of the distal shaft section 250 are largely recoverable and will continues to provide push-back forces (pf) for secured engagement of the electrodes 112 over a long period of time (see FIG. 2(d)). The mechanical properties and behavior of the distal shaft section 850 versus the distal shaft section 250 are further discussed with respect to FIG. 10 below.

When a distal shaft section (e.g., 250) is radially compressed by mechanical swaging, certain material strains and associated material stresses are induced and will decay and relax with time because of different inherent viscoelastic properties of material for various constituent layers (e.g., 210, 220, and 230) of the distal shaft section (e.g., 250). FIG. 10 is an example graph illustrating the relaxation of a compressive material stress (v-axis) that is associated with the decaying of a swaging-induced material strain over time (x-axis) for different polymer materials of the distal shaft section 250, including a hyperelastic rubber material (e.g., a first curve 1001), a thermoplastic elastomer material (e.g., a second curve 1002), and a thermoplastic material (e.g., a third curve 1003), respectively. Each stress relaxation curve may have, or infinitely approach, a plateau or stabilized value at a different rate of stress relaxation. For example, when subjected to an initial compressive material deformation or strain (ε₀), a hyperelastic rubber material can exhibit a quicker stress relaxation than a thermoplastic elastomer material, which in turn shows a faster rate of stress relaxation than a thermoplastic material with time (t). As a result, the hyperelastic rubber material (e.g., rubber members 220) can quickly achieve its steady state with retaining a high plateau value of material stress associated with a minimal compression set (i.e., a high recoverable material strain). By comparing the steady states for the polymer materials of different types subjected to a certain initial material strain (ε₀), the stress relaxation curves (e.g., 1001, 1002, 1003) illustrated in FIG. 10 indicate that the rubber material ( i.e., curve 1001) can well retain a higher compressive stress and strain (in order words, experience less compression set) than the thermoplastic elastomer material (i.e., curve 1002) at a shorter period of time. In contrast, the rigid, thermoplastic polymer or plastic material may only approach a steady or plateau state of stress relaxation towards zero over a very long period of time and can only retain a minimal material stress and strain because of its high compression set of material of >50 to nearly 100%.

Due to the hyperelastic nature of the underlying rubber members 220, the distal shaft section 250 of an elongate shaft 120 can largely retain the swaging-induced material strains and stresses to continuously impart a high push-back force or pressure (pf) onto the electrode 112, under which the swaged electrode tends to be intimately secured via an interference fit (e.g., friction fit) with the distal shaft section 250.

An extent of stress relaxation towards a steady stressed state largely depends on polymer type, and this can be altematively characterized by the inherent compression set of material. For example, a rigid thermoplastic polymer material generally has a very low yield strain (e.g., less than 10%), such that when subjected to a given compressive deformation, the material tends to fail and exhibit 100% compression set (e.g., permanent deformation). In contrast, a thermoplastic elastomer material has a relatively higher yield strain (e.g., greater than 10%), and can undertake relatively high compressive deformation, such that when subjected to a specific compressive deformation, the material is able to attain a steady stressed state and exhibit a finite compression set of less than 100% (i.e., a recoverable material strain upon unloading). In particular, as compared to thermoplastics and thermoplastic elastomer materials, thermosetting elastomers (or simply rubbers) possess characteristic material hyperelasticity without definitive yield strain of material to likely exhibit considerably lower compression sets of <<100% (e.g., 30% or less) with much less temperature dependence, because of material's chemically cross-linked network structure. For example, silicone thermosetting elastomers or silicone rubbers are suitable for applications at high pressures and temperature variations, because of material's excellent hyperelasticity and very low compressions sets that are largely independent of environmental conditions at high temperatures up to 250° C. The suitable rubber material, in particular silicone rubber, can be selected for the one or more rubber members (e.g., 220, 320-322, 420-425) for the distal shaft section 250 of an elongate shaft 120 in view of material's compression set. A lower compression set of the one or more tubular rubber members (e.g., 220, 320-322, 420-425) provides higher hyperelasticity, and higher tendency that the one or more rubber members (e.g., 220, 320-322, 420-425) will be able to continuously retain the higher push-back forces for the distal shaft section 250.

The term "compression set" discussed herein can be understood as follows. Suppose that a (rubber) material is variably loaded to maintain a certain amount of compressive deformation or strain (ε₀) at a specific temperature and then released free after a certain length of time, the compression set of material is measured as the percentage of how much this specific compressive strain (ε₀) would not be able to recover, namely the unrecoverable (i.e., inelastic or viscous) material strain (εᵥ), but be "set" permanently. By definition per ASTM D395, the compression set (c) of a rubber material can be readily characterized in terms of a recoverable or elastic material strain (εₑ) that can be readily measured after the material has been constrained at a constant material strain (ε₀) at a specific temperature for a certain length of time and then released free. That is, *c* = (1 - *εₑ*/*ε*₀) × 100% where ε₀ = εₑ + εᵥ.

The decaying or time-evolving loss of the push-back radial pressure of the shaft 250 against the swaged electrode 112 due to the stress relaxation behavior of the polymeric shaft material(s) would eventually reach, or tend to reach, a steady stressed state as the unrecoverable material strain tends to become "permanently" set after a certain period of time. For securement of the electrode 112 to the distal shaft section 250, such a steady push-back radial pressure of the shaft 250 must be nonzero, but sufficiently high to provide a reliable securement of the electrodes 112 and also a good sealing capacity against any possible fluid migration into the inside of the elongate shaft 120, including the distal shaft section 250. In other words, the shaft material(s) under the fixed, swaging- induced radial compressive strain (*ε*₀) must have a relatively low permanent strain (εᵥ << ε₀), e.g., a lower compression set (c « 100%), such that the shaft material(s) would still be capable of largely recovering or bounce-back to original free states, thus imparting a sufficiently high bounce-back or resilient radial pressure against the electrodes 112.

Referring back to FIG. 10, stress relaxation behaviors under a given compressive deformation at a temperature is qualitatively represented. A rubber material may be able to attain a high, nonzero stress plateau (or the push-back pressure) with a very low compression set, whereas a thermoplastic material relaxes very slowly, and relevant retaining stress (or the push-back pressure) tends to largely disappear over time. Depending on the chemically cross-linked network structures and chemical characteristics, various rubber materials may have different thermophysical stability of material and material resiliencies or compression sets. Accordingly, the rubber members (e.g., 220, 320, 420) may be made of high resilient silicone rubbers because of material's outstanding thermo-physical stability and very low compression sets less than 30% at elevated temperatures up to 250 ^{O}C.

Therefore, for reliable securement of multiple swaged electrodes 112 on the distal shaft section 250 of an elongate shaft 120, the present disclosure selects composite structure made of a combination of rubber material and thermoplastic polymers (including thermoplastic elastomer materials). For example, the one or more rubber members (e.g., 220, 320-322, 420-425) are made of highly resilient elastomeric material(s), e.g., thermosetting silicone rubbers, nitrile butadiene rubbers (NBR), natural rubbers, etc., in combination with the inner and outer thermoplastic layers (e.g., 210 and 230) made of thermoplastic polymers including thermoplastic elastomers, e.g., nylons, polyesters, poly(bisphenol A carbonate), polysulfone, poly(ether imide), poly(ether block amide) copolymers, poly(ether-co-ester) block copolymers, thermoplastic polyurethanes, stvrenic copolymers, thermoplastic olefinic elastomers, etc. The rubber material imparts the elongate shaft assembly 120 with the high material resiliency and hyperelasticity, while the thermoplastic polymers provides the distal shaft section 250 of an elongate shaft 120 ease of manufacturability via thermal fusion bonding or melt reflow.

Molecularly, thermoplastic polymer materials are largely linear polymers comprised of very long polymer chains with or without relatively short branches, whereas thermosetting rubbers (or simply rubbers) are network polymers comprised of chemically cross-linked molecular segments or polymer chains. Because of their differences in polymer structure, rubbers have the outstanding thermo-physical stability of material, and importantly, low compression sets (or high resiliency) at ambient and elevated temperatures of interest (for example, <60° C) during the life cycle of medical devices, such as terminal EO sterilization, thermal cycling for simulating extreme climatic conditions, accelerated aging, etc.

Based on the solid-state structures of material, thermoplastics and thermoplastic elastomers can be classified as semi-crystalline and amorphous polymers, which exhibit the characteristic, critical solid-state thermal transition temperatures of material, e.g., melting temperatures for semi-crystalline polymers or glass transition temperatures for amorphous polymers. Because of such thermally induced thermal transition from a solid to a liquid state, each of thermoplastics and thermoplastic elastomers can be repeatedly shaped or formed at some elevated temperatures above the characteristic, critical thermal transition temperature of material by means of melt processes (e.g., melt extrusion, melt reflow, etc.). However, unlike thermoplastics and thermoplastic elastomers, rubber materials, including silicone rubbers, do not have any melt processability. A rubber material, supplied as a reactive liquid or liquid-like polymer system, can be only shaped or formed once, then followed by the underlying chemical conversion (e.g., curing) into a permanent thermosetting solid rubber material via an underlying cure reaction of the material. Because of its chemically cross-linked structure of material that remains permanent under certain thermal conditions (i.e., thermo-physical stability of material), a rubber material generally has a significantly higher resiliency than a thermoplastic polymer or a thermoplastic elastomer material.

Accordingly, as an example, the elongate tubular inner thermoplastic layer 210 can be made of relatively rigid thermoplastic or thermoplastic elastomer materials, while the outer thermoplastic layer 230, if any, can be made of same as, or softer thermoplastic or thermoplastic elastomer material than, the inner thermoplastic layer 210. The rubber members (e.g., 220, 320, 420) of the distal shaft section 250 are preferably made of a highly resilient, temperature-invariant rubber having a relatively low compression set of less than 30%, preferably less than 10%. Preferably, the rubber members (e.g., 220, 320, 420) are made of silicone rubbers that impart good adhesion to the inner and outer thermoplastic layers (e.g., 210 and 230). In some embodiments, the surfaces of the rubber members (e.g., 220, 320, 420) are chemically activated or treated by means of organosilane coating or plasma treatment. As an example, for the distal shaft section 250 of an elongate shaft 120 shown in FIG. 2, the inner thermoplastic 210 and the outer thermoplastic layer 230 are premade of thermoplastic or thermoplastic elastomer material having a relatively high material hardness (e.g., > Shore 70D; Pebax^{®} 7233SA01, Pebax^{®} 7033SA01, Rilsan^{®} BESNO nylon 11, Rilsan^{®} AESNO nylon 12, Pellethane^{®} 2363-75D, or equivalent) and relatively low material hardness (e.g., Shore D35; Pebax^{®} 3522 SA01, Pellethane^{®} 2363-90AE, etc.) by means of melt extrusion, respectively.

Referring back to FIG. 1 and FIG. 8, the catheters 100 and 800, which are similarly constructed of various shaft assemblies but differ in their respective distal shaft sections 250 and 850 only, are described herein, respectively. For example, like the catheter 800, the catheter 100 can be configured as an elongate electro-anatomic electrophysiology (EP) catheter structurally comprised of at least three shaft sections that are seamlessly integrated as an integral entity via thermal fusion bonding or melt reflow: a proximal shaft section (e.g., 150), a deflectable shaft section (e.g., 200), and a distal shaft section (e.g., 250). The distal shaft section 250 of the catheter 100 can be manufactured by forming and integrating with some other functional shaft sections, such as a tubular moisture-barrier shaft sections 254, and/or a tip-fitting shaft section 253, etc., to result in a distal FSA 270 of the catheter 100.

The proximal shaft section 150 of the catheter 100 is configured to provide column strength and torqueability for a deflectable EP catheter. For this purpose, the proximal shaft section 150 has a tubular composite structure composed of an inner polymer layer, an intermediate braided layer woven of multiple threads of thin metallic wires (e.g., stainless steel) in pattern, and an outer polymer layer. The metallic braided layer is fully embedded between the inner and outer polymer layer extruded of the same or two different thermoplastic polymer materials. For example, the inner and outer polymer layers of the proximal shaft section 150 can be pre-extruded of a thermoplastic polymer material that has a relatively high material durometer of greater than or equal to Shore D70, and then integrated with the metallic braided layer via common thermal fusion bonding or melt reflow techniques that are known of the art.

The deflectable shaft section 200 of the catheter 100 is configured to impart distal deflectability (or steerability) for enabling an elongate catheter 100 to pass through the tortuous vasculature and have desired geometric configurations for easy accesses to the targeted sites within the heart anatomy. Accordingly, the deflectable shaft section 200 includes multiple interconnected tubular shaft sections, each of which may have different column flexibilities or rigidities. To enhance column stability for the purpose of preventing column collapsing when forcibly deflected, the deflectable shaft section 200 may also have a similar composite shaft structure to that of the proximal shaft section 150. Hence, the deflectable shaft section 200 may be considered as a flexible variant or extension to the proximal shaft section 150. In particular, the deflectable shaft section 200, compared to the proximal shaft section 150, similarly comprises an elongate tubular inner polymer layer and an intermediate metallic braided layer, but differently includes an outer polymer layer in multiple interconnected tubular sections made of various chemically compatible thermoplastic elastomer materials with varying material flexibilities or durometers ranging from Shore 25D to Shore 75D. The deflectable shaft section 200 can be structurally integrated with the proximal shaft section 150 by means of reflow or thermal fusion bonding to result in a proximal-deflectable shaft assembly (PDA) of the catheter 100.

The deflectable shaft section 200 has a pull ring (e.g., 261) affixed in position at its distal end. A pair of pull wires (e.g., 262, 263) welded onto the pull ring are properly strung through the internal channels of the PDA to be coupled with the manual deflecting mechanism residing within the handle (e.g., 110).

As an integral assembly, the premade PDA can have the intermediate metallic braided layer protrude beyond its distal end for some distance. This extended metallic braided layer can be disposed beneath the inner thermoplastic layer 210 but over the rubber member(s) 220 to form a distal shaft section 250. As such, the distal shaft section 250 can have an unbraided portion (e.g., 251) be distally integrated or interconnected with other unbraided tubular sections, such as a moisture-barrier tubular section 254, a tip-fitting tubular section 253, etc., to result in a distal FSA 270. On the other hand, the distal shaft section 250 can have a braided portion (e.g., 252) to impart a good structural continuity and smooth transition proximally with the pre-made PDA (e.g., 150 and 200).

The distal FSA 270 of the catheter 100 for an electro-anatomical contact force, irrigation RF ablation catheter (e.g., Tacticath^{®} SE or Tactiflex^{®} SE contact force irrigation ablation catheter) provides a tubular shaft section for fitting to an ablation electrode tip (e.g., the tip-fitting shaft section 253), through which the tip functional assembly (TFA) can be inserted and disposed into a center lumen (e.g., 265A) of the distal FSA 270. The ablation tip can be adhesively affixed in position into the tip-fitting shaft section 253 of the distal FSA 270. For example, the tip-fitting shaft section 253 is configured to couple the TFA comprising multiple functional elements as required for accomplishing advanced EP therapeutic and/or diagnostic procedures, and may include a tip (ablation) electrode, an irrigation tube, a thermocouple, an optical force sensor, a magnetic positioning sensor, and other accessory components (e.g., potting adhesive, magnetic sensor cage, conductors, etc.).

Also, the distal FSA 270 of the catheter 100 may also provide a highly flexible, moisture-barrier tubular shaft section 254, which circumferentially encapsulates an optical force sensor disposed within the center lumen 265A of the distal FSA 270 and prevents any penetration of moisture through the moisture-barrier shaft section 254. These tip-fitting shaft section 253 and the moisture-barrier tubular shaft section 254 are located and structurally integrated together with the distal shaft section 250) by means of thermal fusion bonding or melt reflow.

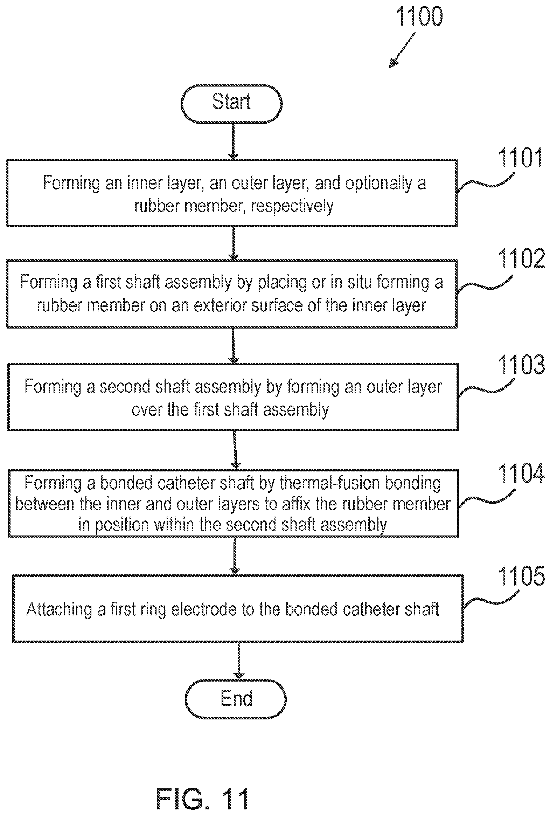

FIG. 11 is a flow chart of an example method 1100 for manufacturing a catheter shaft. The method 1100 can be implemented in following steps 1101, 1102, 1103, and 1104. Step 1101 involves forming an inner layer, an outer layer, and optionally a rubber member, respectively. In many embodiments, the inner layer is an inner thermoplastic layer having an elongated tubular shape, which can be formed by melt (or tubing) extrusion. Similarly, the outer layer may have an elongated tubular shape, which may be formed by melt or tubing extrusion. In many other embodiments, a rubber member is formed of a (liquid or paste-like) rubber compound, where the rubber compound is shaped by a mold and then thermally press-cured within the hot mold at an elevated cure temperature for a duration of time.

Step 1102 involves placing an inner layer (over a mandrel) and then forming a first shaft assembly by placing a rubber member on an exterior surface of the inner layer. In some embodiments, part of step 1101 (i.e., forming a rubber member) may be delayed until step 1102, in which forming a first shaft assembly involves *in situ* forming a rubber member that is intimately bondable to the exterior surface of the inner layer. Further in some embodiments, step 1101 for forming the rubber member or step 1102 for in situ forming the rubber member for the first shaft assembly involves post-curing at a predetermined temperature for a specified time period in an effort to optimally enhance material resiliency or hyperelasticity of the rubber member. For example, post-curing of the rubber member formed (or in situ formed) of a silicone rubber compound involves heating the "standalone" rubber member (or the first shaft assembly) at a temperature between 110° C to 180° C for a time period between 1 hours to 4 hours. In many embodiments, the rubber member as formed in step 1101 or in situ formed in step 1102 may be surface treated to improve its chemical compatibility with the inner and/or outer layers.

In some embodiments, the rubber member includes a tubular rubber member (e.g., 220 in FIG. 2 or 720-711 in FIG. 7) that encircles and extends along a first length of the inner thermoplastic layer (e.g., 210 in FIG. 2). In some embodiments, one or more tubular rubber members (e.g., 220 in FIG. 2 or 720-711 in FIG. 7) may be longitudinally spaced on the exterior surface of the inner thermoplastic layer (e.g., 210). In some embodiments, forming the first shaft assembly involves placing, or in situ forming, a plurality of discrete rubber members (e.g., a group or set of rubber members 320-322 in FIG. 3 or 420-425 in FIG. 4) on the exterior surface of the inner thermoplastic layer (e.g., 210) so that the discrete rubber members are circumferentially distributed and spaced apart along the inner thermoplastic layer. In some embodiments, at least one of the plurality of discrete rubber members comprises protruding portions (e.g., the protruding portions 401 and 402 in FIG. 4) that protrude circumferentially from a perimeter of the discrete rubber member. Each extension is configured to enhance coupling of the discrete rubber member with at least one of the outer layer and the inner thermoplastic layer.

Step 1103 involves forming a second shaft assembly by placing an outer layer over the first shaft assembly. In many embodiments, the outer layer and/or the inner layer are made of same or different thermoplastic material. For example, the thermoplastic material of the outer layer and/or the inner thermoplastic layer has a durometer of greater than Shore D40, and the range of Shore D60 to D85. In some embodiments, the outer thermoplastic layer has a different durometer from that of the inner thermoplastic layer and preferably has a durometer of less than Shore D60. The one or more rubber members have a durometer equal to or less than Shore A65 (e.g., silicone rubber). As per the examples discussed with respect to FIGS 2-4, and FIG. 7, the inner layer 210 and the outer layer 230 can be formed of same or different thermoplastic material, the one or more rubber members (e.g., 220, 320-322, 420-425, or 720-722) can be formed and then disposed, or *in situ* formed, on exterior surface of the inner layer 210, and the outer layer 230 can be disposed over the rubber members. Furthermore, the inner layer 210, the rubber members 220, and the outer layer 230 can be made integral by thermal fusion bonding e.g., by melting and reflowing the inner and outer thermoplastic layers.

In many embodiments, the step 1102 of forming the first shaft assembly further involves applying an adhesive to temporarily affix the rubber members (that are pre-formed in step 1101) onto the exterior surface of the inner thermoplastic layer. Preferably the adhesive when cured is thermoplastic in nature. For example, the adhesive comprises cyanoacrylate adhesives based on a cyanoacrylate monomer or oligomer.

In many embodiments, the step 1104 of forming the catheter shaft involves thermal-fusion bonding process to form an integral shaft. For example, an integral catheter shaft can be formed by melting and reflowing the inner thermoplastic layer and the outer thermoplastic layer of the second shaft assembly under radial compressive pressure. In some embodiments, the heating involves placing a heat-shrinkable tube over the outer layer to apply a temperature in a predetermined temperature range (e.g., 200° to 250° C).

Step 1105 involves attaching one or more ring electrodes (e.g., a first ring electrode) to bonded catheter shaft (e.g., by mechanical swaging) so that each of the ring electrodes induces a radial hyperelastic deformation of the rubber members to inhibit loss of an interference fit between the first ring electrode and the catheter shaft. For example, as discussed with respect to FIGS 2-4, and FIG. 7, the electrodes 112 can be attached to the exterior surface of the outer layer 230 by mechanical swagging that induces radial elastic deformation of the rubber members (e.g., 220, 320-322, 420-425, or 720-722).

In many embodiments, attaching the one or more electrodes during subsequent assembling process of the catheter 100 involves swagging the one or more electrodes onto the distal shaft section 250 of the catheter 100 to induce radial hyperelastic or recoverable deformation of the one or more rubber members while the one or more rubber members induces a push-back pressure against the one more or electrodes to securely retain an effective interfere or friction fit of the electrode(s) 112 onto the distal shaft section 250 for a prolonged period of time.

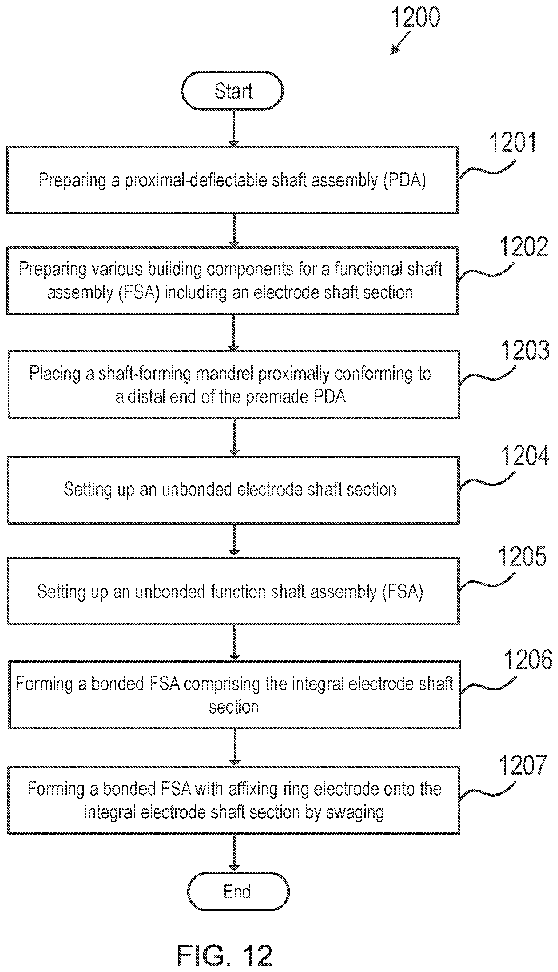

In some embodiments, the method 1100 can be further integrated into a manufacturing process of a catheter (e.g., 100) that includes additional shaft sections. For example, the catheter 100 including the distal FSA 270 (e.g., comprising a distal shaft section 250) can be manufactured and adherently interconnected with a pre-made PDA (e.g., 150 and 200) as an integral entity, by means of thermal fusion bonding (e.g., melt reflow). FIG. 12 is an illustrative flow chart of an example method 1200 for manufacturing the catheter 100 including the distal FSA 270 comprising an integral distal shaft section 250 on which electrodes are to be mounted. Method 1200 starts with two preparation steps, including step 1201: preparing the pre-made PDA (e.g., 150 and 200) for manufacturing a distal FSA (e.g., 270) and step 1202: preparing various building components of the distal FSA (e.g., 270) including a distal shaft section (e.g., 250) on which electrodes are to be mounted.

In some embodiments, step 1201 may involve coupling a pair of pull wires (e.g., 262 and 263) to the pull-ring 261 by laser welding, and then disposing the pull wires assembly through the center lumen of the pre-made PDA (e.g., 200 and 150). The pull ring 261 can be situated inside and adhesively affixed to the distal end of an integral, premade PDA, as illustrated in FIGS. 7 and 9. In addition, an intermediate metallic braided wire distally extending from the PDA (e.g., 213) can be stripped and cleaned by means of laser ablation or other chemical and/or physical techniques, and then cut in a length equal to the braided shaft portion 252 (e.g., in FIG. 7) of the distal shaft section 250.

In some embodiments, at step 1202, the inner thermoplastic layer 210 of the distal shaft section 250 may be pre-extruded of a relatively rigid thermoplastic polymer or thermoplastic elastomer material in a continuous tubing form and cut in tubular sections, each having a length equal to the length of the distal shaft section 250. Similarly, the outer thermoplastic layer 230 of the distal shaft section 250 may be pre-extruded of the same as, or relatively softer than, thermoplastic polymer or thermoplastic elastomer material than, that of the inner layer 210 in a continuous tubing form and cut in tubular sections, each having a length equal to, or slightly less than, a length comparable to that of the distal shaft section 250. The rubber member(s) 230 may be pre-made of a reactive liquid/paste-like rubber compound via liquid/paste rubber extrusion, reactive injection molding, reactive compression molding, or liquid casting, etc. commonly known of the art for rubber processing. Post-curing the rubber member(s) at a proper high cure temperature may be necessary for enhancing material resiliency and hyperelasticity.

The method 1200 further utilizes a non-stick PTFE-coated shaft-forming mandrel proximally conforming to the distal end of a premade PDA. The proximal end of such a shaft-forming mandrel has an outer diameter conforming to the inner diameter of the center lumen of the PDA at the distal end of the PDA where the pull ring 216 is adhesively affixed.

Accordingly, method 1200 further includes step 1203 that involves placing the shaft-forming mandrel, by which the proximal portion of the mandrel is inserted and snugly fitted to the center lumen of the PDA until the proximal end of the mandrel is snugly in contact with the pull ring 261 of the PDA (e.g., 150 and 200).

To make the distal FSA 270 comprising the distal shaft section 250 that is intimately adhered to and integrated with the premade PDA of the catheter 100, method 1200 can include multiple steps e.g., steps 1204 to 1207.

Step 1204 involves setting up or assembling an unbonded distal shaft section 250, during which the inner thermoplastic layer (e.g., 210) is disposed over the shaft-forming mandrel, optionally followed by disposing the pre-stripped or bare metallic braid layer (e.g., 213). The groups of the rubber member(s) 220, each in specified sizes, along with the outer thermoplastic layer 230, are sequentially introduced and disposed over the metallic braided layer (e.g., 213) or over the inner layer (e.g., 210).

Step 1205 involves setting up an unbonded FSA. For example, a premade moisture-barrier tubular section and the tip-fitting tubular section can be longitudinally inserted over the non-stick mandrel, one by one, to attach to the distal end of the unbonded distal shaft section 250, thus resulting in the unbonded distal FSA 270. The unbonded distal FSA 270 is then fully encapsulated by a high-temperature heat shrink tube (e.g., PTFE heat shrink tube).

Step 1206 involves forming a bonded FSA 270 comprising the integral distal shaft section 250 that is also adherently interconnected with the pre-made PDA (e.g., 150 and 200) by means of thermal fusion bonding or melt reflow at a high temperature (e.g., >180 to 250 °C) in a convective oven. Oven heating will make all the thermoplastic polymer materials of the unbonded FSA 270 (including the inner & outer thermoplastic layers (e.g., 210) of the unbonded distal shaft section 250, the moisture-barrier tubular section 254, and the tip-fitting shaft section 253, etc.,) melt and reflow to fill up any unoccupied spaces within the distal FSA 270 under radial inwards pressure exerted by the outmost heat shrink tube, thereby forming the bonded distal FSA 270 comprising the integral distal shaft section 250 that is in turn, adherently interconnected with the premade PDA as an elongate catheter shaft 120.

After thermal fusion bonding or melt reflow, step 1207 involves finishing the bonded FSA 270 for subsequent assembling of the catheter 100. For example, the heat shrink tube is removed from the resultant FSA 270, followed by placing ring electrodes 112 onto the integral distal shaft section 250 and inserting the TFA into the tip-fitting shaft section 253, stringing various elongate components coupled to the ring electrodes 112 (e.g., conductive wires 855) and to the TFA (e.g., conductive wires of sensors and thermocouple wires, irrigation tube, etc.) through the center lumen of the integral, interconnected catheter shaft 120, adhesively attaching the TFA to the tip-fitting section 253 of the distal FSA 270, and affixing the TFA in position within the center lumen of the distal FSA 270 by applying a potting adhesive through the adhesive-potting holes 700, etc. Finally, the ring electrodes 112 are affixed onto the integral distal shaft section 250 in position by mechanical swaging.

According to the above embodiments, relevant manufacturing methods for various shaft designs for the distal shaft section 250 of the FSA 270 of catheter 100 is further discussed with some examples as follows:

### Example #1:

For the shaft designs shown in FIG. 3, FIG. 4 and FIG. 7, the inner thermoplastic layer 210 pre-extruded of nylon 12 (or PA12) is introduced onto a shaft-forming mandrel. Optionally, the layer 210 is at least partially covered by the braided metallic layer 213 extending from the premade integral PDA (see FIG. 8). Then, a plurality of the rubber members (e.g., 320-322 or 420-425) press-made of a silicone rubber compound (e.g., Momentive Tufel III 92656) and various tubular sections of the outer thermoplastic layer 230 pre-extruded of Pebax^{®} 3533 SA01 PEBA copolymer resin are alternately applied over the metallic braided layer 213 (FIG. 7) or the inner thermoplastic layer 210. Herein, the plurality of arc-like rubber members (e.g., discrete rubber members 320-322 or 420-425) are circumferentially and evenly disposed over the inner thermoplastic layer 210 or the metallic braided layer 213, and temporarily affixed in position by sparingly using an instant cyanoacrylate (CA) adhesive (e.g., Loctite 4011). The resultant, unbonded distal shaft section 250, along with other thermoplastic functional shaft components (e.g., tip-fitting shaft section 253, moisture-barrier shaft section 254, etc.) of the distal FSA 270, is circumferentially covered by a high- temperature heat-shrinkable tube (e.g., PTFE heat shrink tube), and then heated to a high temperature of about 220° C to 250° C for about 10 to 15 minutes, thus forming a bonded distal FSA comprising the integral distal shaft section 250. During heating that effects the thermal fusion bonding or melt reflow within all the thermoplastic components of the distal shaft section 250, all the rubber members (e.g., the discrete rubber members 320-322 or 420-425) would largely remain geometrically intact because of material's inherent chemically cross-linked network structure or thermosetting, while the cured CA instant adhesive (for temporarily affixing the rubber members) would be able to melt and mix with the polymer melts, thus imparting melt adhesion of the rubber members with the rest of thermoplastic polymers (e.g., the inner thermoplastic (PA12) layer 210 and the outer thermoplastic (Pebax 3533 SA01) layer 230) within the distal shaft section 250. The cured CA adhesive is chemically comprised of linear poly(ethyl cyanoacrylate), which is a typical amorphous thermoplastic polymer having a critical solid-state thermal transition or glass transition temperature of about 125 ^{O}C. During heating, the cured CA adhesive is able to reflow or be reshaped, while without affecting the formation of the integral distal shaft section 250 comprising various rubber members 220.

In many embodiments, the highly resilient rubber members are premade of a silicone rubber compound (e.g., Momentive Tufel III 92656) by means of an applicable reactive liquid process (e.g., reactive liquid extrusion, reactive liquid injection molding, reactive liquid casting, etc.), which includes a process step of oven/press curing (e.g., at 177 ^{O}C for 10 minutes). A post-forming thermal cure process, e.g., post-cure, at 177 ^{O}C for 2 hours, may be optionally performed to improve crosslink density of material and thus enhance material's resiliency or hyperelasticity (e.g.,the push-back forces) and optimally minimize material's compression set. The resultant silicone rubber material used for the rubber members has a material durometer of Shore A65, and a very low compression set of about 14% as tested in accordance with ASTM D-395.

To facilitate the heating-induced thermal fusion bonding of the two polyamide-based thermoplastic polymers (e.g., nylon 12 and Pebax 3533 SA01) for the inner and outer thermoplastic layers (e.g., 210 and 230), all the rubber members (e.g., discrete rubber members 320-322 or 420-425) as formed above are chemically pre-treated to impart chemical compatibility with polyamides (e.g., nylon 12 and Pebax 3533SA01 PEBA copolymer) by either conventional physical methods known in the art (e.g., plasma surface treatment, etc.) or by a chemical method with use of a diluted coating solution comprising one or more organosilane coupling agents at a concentrationof about 2 to 5%. Relevant technology & processes for organosilane treatment of silicone rubber in improving material's chemical compatibility with thermoplastic polymers like nylons, PEBAs, and TPUs, etc., are described in *"Silane Coupling Agents, Connecting Across Boundaries"* Gelest Inc., 2014], and US Patent Application No. 20210162200. The organosilane coupling agents here include, but not limited to, various commercially available amino- or amine-containing silanes, such as 3-*aminopropyl* trimethoxysilane, 3-aminopropyl triethoxysilane, 4-aminobutyl triethoxysilane, N-phenylaminomethyl triethoxysilane, 4-amino-3,3-dimethylbutyl trimethoxysilane, N-(2-aminoethyl)-3-aminopropyl trimethoxysilane, etc. That is, the pre-made rubber members (e.g., discrete rubber members 320-322 or 420-425) are briefly dipped or immersed into an effective coating solution comprising one or more active organosilane coupling agent(s) and let the coating fully dry or cured under ambient or elevated temperatures (at optimal humidity of 50 to 60%) via the so-called hydrolysis-condensation cure mechanism as known in silicone chemistry. The resultant coating for the rubber members (e.g., discrete rubber members 320-322 or 420-425) would not only exhibit adherences to the silicone rubber substrates, but also have the activated silicone surfaces rich in active amino or amine groups, which exhibit inherent molecular affinity to polyamides or nylons, thermoplastic polyurethanes, and the like, for thermal fusion bonding by these thermoplastic polymer materials.

The portion 750A, 750B, and/or 750C of the distal shaft section 250, where electrode(s) is to be mounted (in FIGS 7), may have a very thin outer thermoplastic polymer skin, as part of the outer thermoplastic layer 230 that may have "reflowed" from the other portion 280 of the distal shaft section 250 where electrode(s) are not present. When present and bonded to the exterior surface of the rubber members, such a skin-like outer thermoplastic layer 230 would not noticeably affect radial compression of the rubber members 220 and impose any noticeable physical resistance to the hyperelastic bounce of the rubber members 220, either. That is, under the radial compressive pressures exerted by a swaged ring electrode, this will allow the rubber members with the high hyperelastic deformability to be able to actively take the very high radial compressive deformations while without the occurrence of material yielding, and then attain a steady push-back forces against the swaged electrodes for reliable securement and sealing of the swaged electrodes, because of the unique stress relaxation characteristics and very low compression sets (at about 14%) of silicone rubber.

As compared to the shaft design shown in FIG. 3, the shaft design shown in FIG. 4 with two protruding portions (e.g., 401, 402) at two sides may likely provide relatively high push-back pressures and forces against the swaged electrodes, because the side protrusions have been twisted by swaging and would tend to energetically bounce back.

### Example #2

For the shaft design shown in FIG. 2, the inner thermoplastic layer 210 and the outer thermoplastic layer 230 are premade of thermoplastic polymers having a relatively high material hardness (e.g., Shore 72D; Pebax 7233SA01) and a relatively low material hardness (e.g., Shore D35;Pebax 3522 SA01) by means of melt extrusion, respectively. A single tubular rubber member is premade of a reactive silicone rubber compound (e.g., Momentive Tufel III 92656) by means of a reactive liquid extrusion process and optionally a post-forming thermal cure process, as detailed in Example #1.

To manufacture the portion 250A of the distal shaft section 250 (in FIG. 2), the inner thermoplastic layer 210, cut in a length equal to that of the entire distal shaft section 250, is introduced to snugly fit onto a shaft-forming mandrel. Optionally, the layer 210 is partially covered by the metallic braided layer 213 extending from the premade, integral PDA (see FIG. 7). Then, the tubular rubber member 220 cut in a length somewhat less than that of the distal shaft section 250 is centered about the distal shaft section 250, and then directly disposed over the inner thermoplastic layer 210 or the metallic braided layer 213. Further, the outer thermoplastic layer 230, cut in a length equal to that of the distal shaft section 250, is disposed over the tubular rubber member 220. By heating, the resultant nonbonded shaft assembly for the distal shaft section 250 at a high temperature of about 200° C to 240° C for 10 to 20 minutes, the inner and outer thermoplastic layers 210 and 230 will melt to reflow and fully lock the tubular rubber member in position between the bonded layers 210 and 230, thereby forming the integral distal shaft section 250 of the catheter 100.

In some embodiments, a singular tubular rubber member 220 may include one or more longitudinally disposed slots or holes die-cut through the wall of the tubular rubber member 220 to assure melt flow into any unoccupied spaces that may exist between the inner thermoplastic layer 210 and the tubular rubber member 220. The tubular rubber member 220 is not melt-processable or re-shapable, thus the slots or holes enhance the intimate thermal-fusion bonding of the inner and outer thermoplastic layers (i.e., 210 and 230) across and with the tubular rubber member 220.

Similar to the above example #1, to impart the intimate layer-to-layer adherence via thermal fuse bonding or melt reflow, exterior surfaces of the inner thermoplastic layer 210 and the tubular rubber member 220 may be siliconized and chemically activated, respectively, by using the same or similar coating solution comprising one or more amino/amine-containing organosilane coupling agents at a concentration of about 2 to 5% prior to the above shaft assembling process. To enhance the layer-to-layer adherence between the tubular rubber member 220 and the inner thermoplastic layer 210 (e.g., made of Pebax 7233 SA01) layer or the outer layer 230 (e.g., made of Pebax 3533), a thin film layer of an instant CA adhesive (e.g., Loctite 4011) may be applied in between.

### Example #3

In some embodiments, to simplify and facilitate relevant manufacturing processes of the distal shaft section 250, the rubber members/layer as shown in FIGS. 2-4 can be pre-formed over the pre-formed inner thermoplastic layer 210 as a liner, thereby forming the so-called integral liner-gasket entity. The inner thermoplastic layer 210 (or the liner) is made of a relatively rigid thermoplastic polymer material, whose solid-state thermal transition temperature of material (e.g., melting temperature for semi-crystalline thermoplastic polymer or glass transition temperature for amorphous thermoplastic polymer) is considerably higher than the applicable cure temperature (e.g., 110° C to 170° C) for typical silicone rubber compounds. For instance, the inner thermoplastic layer 210 is melt-extruded of nylon 6 (e.g., Zytel 7301 NC010), whose characteristic melting temperature is about 221° C. The pre-formed inner thermoplastic layer 210 is continuously fed through an extrusion die as the liner, onto which the rubber members/layer are shaped in the desired geometric profiles, followed by an *in situ* curing to result in an intimately bonded liner-gasket entity, via a reactive liquid/paste extrusion process with use of a self-adhesion silicone rubber compound, e.g., Silastic RBL-9694-30P. To optimally enhance crosslink density and minimize the permanent compression set for the *in situ* cured silicone rubber material, a post-forming curing can be optionally conducted at elevated temperature of up to 170 ^{O}C for about two hours, while without any thermo-physical compromise on the solid-state structural integrity of the so-formed integral liner-gasket entity. In addition, to improve the melt adhesion of the outer thermoplastic layer 230 (which may be pre-extruded of a relatively soft polymer material (e.g., Pebax 3533 SA01) with the integral liner-gasket entity, similar chemical treatment on the integral liner-gasket entity comprising the silicone rubber member/layer, as detailed above, can be carried out because the cured silicone rubber material within the integral liner-gasket entity may no longer exhibit any adhesion properties with other thermoplastic materials (e.g., an outer thermoplastic layer 230, tip-fitting shaft section 253, moisture-barrier shaft section 254, etc.).

Then, the resultant shaft assembly (i.e., the distal shaft section 250) comprising an integral liner-gasket (or an integral inner thermoplastic layer-tubular rubber member) entity, along with other functional shaft sections (e.g., tip-fitting shaft section 253, moisture-barrier shaft section 254, etc.), is fully enclosed by a high- temperature heat shrink tube (e.g., PTFE heat shrink tube), and heated to high temperature of about 270° C by means of induction heating. As such, the inner and outer thermoplastic layers 210 and 230, along with other functional shaft sections (e.g., 253 and 254), will melt and redistribute to result in the intimate adherences between different polymer materials upon cooling.

FIG. 13 is a schematic and block diagram view of a system 10 that can be used for ablation (e.g., RF ablation or pulsed field ablation) to destroy abnormal cardiac tissue within the heart anatomy. In particular, system 10 may be used for electroporation-induced primary necrosis therapy, which refers to the effects of delivering electrical current in such manner as to directly cause an irreversible loss of plasma membrane (cell wall) integrity leading to its breakdown and cell necrosis. This mechanism of cell death may be viewed as an "outside-in" process, meaning that the disruption of the outside wall of the cell causes detrimental effects to the inside of the cell. Typically, for classical plasma membrane electroporation, electric current is delivered as a pulsed electric field (*i.e.,* pulsed field ablation (PFA)) in the form of short-duration pulses (e.g., 0.1 to 20 ms duration) between closely spaced electrodes capable of delivering an electric field strength of about 0.1 to 1.0 kV/cm.

The system 10 includes a catheter (e.g., 100) having an elongate shaft (e.g., 120 including a distal shaft section 250 with one or more rubber members) coupled to a handle assembly 110. A connector 130 may be coupled at a proximal end of the handle 110 to provide mechanical and electrical connection(s) for cable 56 extending from an ablation generator 26. The connector 130 may comprise conventional components known in the art and as shown is disposed at the proximal end of the catheter 100. The catheter 100 may also include other conventional components not illustrated herein such as a temperature sensor, additional electrodes, and corresponding conductors or leads disposable via the elongate shaft 120.

In some embodiments, the catheter 100 includes a diagnostic and/or therapeutic shaft assembly (i.e., FSA 270) attached to the distal end portion 201A of the deflectable shaft section 200. The diagnostic and/or therapeutic shaft assembly comprising an electrode shaft section can have any suitable configuration for performing a diagnostic and/or therapeutic medical procedure. For example, in some embodiments, the diagnostic and/or therapeutic shaft assembly (i.e., FSA 270) includes an electrode shaft section 250 with the electrodes 112 configured to accomplish a diagnostic and/or therapeutic medical procedure (e.g., the electrode shaft section 250). For example, the diagnostic and/or therapeutic shaft assembly comprising the electrode shaft section (e.g., 250) can include electrodes 112 that are electrically coupled to the generator 26 via suitable electrical wire or other suitable electrical conductors extending through the elongate shaft 120.

The electrodes 112 may be used for a variety of diagnostic and therapeutic purposes including, for example and without limitation, cardiac mapping and/or ablation (e.g., IRE ablation). For example, and in some embodiments, the distal shaft portion 250 with the electrodes 112 may be configured as a bipolar electrode assembly for use in bipolar-based electroporation therapy. Specifically, electrodes 112 are individually electrically coupled to the generator 26 (e.g., via suitable electrical wire or other suitable electrical conductors extending through catheter shaft 200) and are configured to be selectively energized (e.g., by the generator 26 and/or computer system 32) with opposite polarities to generate a potential and corresponding electric field therebetween, for PFA therapy. That is, one of electrodes 112 is configured to function as a cathode, and the other is configured to function as an anode. Electrodes 112 may be any suitable electroporation electrodes. In the exemplary embodiment, electrodes 112 are ring electrodes. Electrodes 112 may have any other shape or configuration. It is realized that the shape, size, and/or configuration of electrodes 112 may impact various parameters of the applied electroporation therapy. For example, increasing the surface area of one or both electrodes 112 may reduce the applied voltage needed to cause the same level of tissue destruction. Further, the electrodes 112 on the distal shaft section 250 may be configured as a bipolar electrode assembly. In some embodiments, electrode assembly 100 may configured as a monopolar electrode assembly and use a patch electrode (e.g., return electrode 18) as a return or indifferent electrode.

In some embodiments, the catheter 100 may be configured as an introducer that includes a lumen configured to accommodate insertion and advancement of a diagnostic and/or therapeutic catheter to a target site within a patient's vasculature. The diagnostic and/or therapeutic catheter can be configured for use in any suitable medical procedure such as, for example, cardiac mapping and/or ablation (e.g., PFA).

The handle 110 is configured to be held by a clinician and operable to articulate the deflectable section 200 of the catheter shaft 120. The handle 110 includes a pull wire actuation mechanism that is drivingly coupled with the deflectable shaft section 200 via two pull wires (also referred as deflection wires) affixed onto a pull ring 261 disposed at the distal end of the deflectable shaft section 200. The pull wire actuation mechanism includes an input element that is articulable by the clinician to articulate the pull wires to selectively curve the deflectable shaft section 200. The handle 110 can be further configured to vary the shape, size, and/or orientation of another portion of the catheter 100 other than the deflectable shaft section, such as the electrode shaft section 250. The handle 110 can have any suitable configuration, such as configurations that are conventional in the art.

A plurality of return electrodes designated 18, 20, and 21, which are diagrammatic of the body connections that may be used by the various sub-systems included in the overall system 10, such as an electroporation generator 26, an electrophysiology (EP) monitor such as an ECG monitor 28, a localization and navigation system 30 for visualization, mapping and navigation of internal body structures. In the illustrated embodiment, return electrodes 18, 20, and 21 are patch electrodes. It should be understood that the illustration of a single patch electrode is diagrammatic only (for clarity) and that such sub-systems to which these patch electrodes are connected may, and typically will, include more than one patch (body surface) electrode. The system 1400 may further include a main computer system 32 (including an electronic control unit 50 and data storage-memory 52), which may be integrated with system 30 provided for visualization, mapping and navigation of internal body structures in certain embodiments. The computer system 32 may further include conventional interface components, such as various user input/output mechanisms 34a and a display 34b, among other components.

The generator 26 may be configured to energize the electrode element(s) in accordance with a RF ablation or an electroporation energization strategy, which may be predetermined or may be user-selectable. For electroporation, a variable impedance 27 allows the impedance of the system to be varied to limit arcing from the catheter electrode of catheter (e.g., 100). Moreover, variable impedance 27 may be used to change one or more characteristics, such as amplitude, duration, pulse shape, and the like, of an output of the generator 26. Although illustrated as a separate component, variable impedance 27 may be incorporated in the catheter 100 or generator 26. In some embodiments, each variable impedance 27 may be connected to a different catheter electrode or group of catheter electrodes to allow the impedance through each catheter electrode or group of catheter electrodes to be separately varied. Additional details of an example electroporation systems are discussed in a PCT publication no. WO2018102376A1.

It is to be understood that terms such as "distal," "proximal," "top," "bottom," "front," "side," "length," "inner," and the like that can be used herein merely describe points of reference and do not necessarily limit embodiments of the present disclosure to any particular orientation or configuration. As used herein, "proximal" refers to a direction toward the end of the catheter near the clinician and "distal" refers to a direction away from the clinician and (generally) inside the body of a patient. Furthermore, terms such as "first," "second," "third," etc., merely identify one of a number of portions, components, steps, operations, functions, and/or points of reference as disclosed herein, and likewise do not necessarily limit embodiments of the present disclosure to any particular configuration or orientation.

The terms "longitudinal," "axial" or "axially" are generally longitudinal as used herein to describe the relative position related to a catheter, a catheter handle, or other components of the system herein. For example, "longitudinal" or "axial" indicates an axis passing along a center of a catheter from a proximal end to a distal end, or along a center of the catheter handle from a proximal end to a distal end. The term "radial" generally refers to a direction perpendicular to the "axial" direction.

Disjunctive language such as the phrase "at least one of X, Y, or Z," unless specifically stated otherwise, is intended to be understood within the context as used in general to present that an item, term, etc., may be either X, Y, or Z, or any combination thereof (e.g., X, Y, and/or Z). Thus, such disjunctive language is not generally intended to, and should not, imply that certain embodiments require at least one of X, at least one of Y, or at least one of Z to each be present.

While certain embodiments have been described, these embodiments have been presented by way of example only and are not intended to limit the scope of the present disclosures. Indeed, the novel methods, apparatuses and systems described herein can be embodied in a variety of other forms; furthermore, various omissions, substitutions and changes in the form of the methods, apparatuses and systems described herein can be made. The invention is defined by the following claims.

## Claims

1. A catheter shaft assembly comprising:
an elongate tubular inner layer (210);
a first rubber member (320) disposed on an exterior surface of the elongate tubular inner layer (210);
an elongate outer layer (230) that covers an exterior surface of the first rubber member (320) or the elongate tubular inner layer (210); and
one or more ring electrodes (112) attached to and encircling the elongate outer layer (230) or the first rubber member (320), wherein each of the ring electrodes (112) induces a radial hyperelastic deformation of the first rubber member (320) that inhibits loss of an interference fit between the ring electrode (112) and the elongate outer layer (230) or the first rubber member (320).

2. The catheter shaft assembly of claim 1, wherein the elongate tubular inner layer (210) has the same length as the elongate outer layer (230), or
wherein the first rubber member (320) is elongated, and has a length equal to or shorter than the elongate tubular inner layer (210) or the elongate outer layer (230); and wherein the first rubber member (320) is disposed between the elongate tubular inner layer (210) and the elongate outer layer (230).

3. The catheter shaft assembly of claim 1, further comprising a second rubber member (321);
wherein each of the first rubber member (320) and the second rubber member (321) has a length equal to or longer than a ring electrode (112) of the one or more ring electrodes (112); and
wherein the first rubber member (320) is longitudinally spaced apart from the second rubber member (321) along the elongate tubular inner layer (210),
optionally further comprising a third rubber member (322) having a length equal to or longer than a ring electrode (112) of the one or more ring electrodes (112),
wherein the first (320), second (321), and third (322) rubber members are longitudinally spaced apart from each other at equal distances or varying distances

4. The catheter shaft assembly of claim 1, wherein the first rubber member (320) encircles and extends along a first length of the elongate tubular inner layer (210).

5. The catheter shaft assembly of claim 4, further comprising a second rubber member (321) and a third rubber member (322),
wherein the first rubber member (320), the second rubber member (321), and the third rubber member (322) are circumferentially distributed and spaced apart along the elongate tubular inner layer (210); and
wherein each of the one or more ring electrodes (112) induces a radial hyperelastic deformation on each of the first rubber member (320), the second rubber member (321), and the third rubber member (322) that inhibits loss of the interference fit between each of the ring electrodes (112) and the elongate outer layer (230), the first rubber member (320), the second rubber member (321), or the third rubber member (322).

6. The catheter shaft assembly of claim 5,
wherein at least one of the first rubber member (320) and the second rubber member (321) comprises a protruding portion shaped to enhance coupling with the elongate outer layer (230), or
further comprising a fourth rubber member (423), a fifth rubber member (424), and a sixth rubber member (425), wherein:
the first rubber member (320), the second rubber member (321), the third rubber member (322), the fourth rubber member (423), the fifth rubber member (424), and the sixth rubber member (425) are circumferentially distributed and spaced apart along the first length of the elongate tubular inner layer (210); and
each of the one or more ring electrodes (112) induces a radial hyperelastic deformation of each of the first rubber member (320), the second rubber member (321), the third rubber member (322), the fourth rubber member (423), the fifth rubber member (424), and the sixth rubber member (425) that inhibits loss of the interference fit between the one or more ring electrodes (112) and the elongate outer layer (230), the first rubber member (320), the second rubber member (321), the third rubber member (322), the fourth rubber member (423), the fifth rubber member (424), or the sixth rubber member (425).

7. The catheter shaft assembly of any one of claim 1 through claim 6, wherein the first rubber member (320) is a tubular rubber member comprising at least one longitudinally-oriented through slot (225) or holes (227) for facilitating melt flow-induced space filling and thermal fusion bonding of the elongate tubular inner layer (210), the elongate outer layer (230), and the first rubber member (320), or
wherein the first rubber member (320) comprises a protruding portion shaped to enhance coupling with the elongate outer layer (230) or the elongate tubular inner layer (210), or
further comprising a first electrode wire (855) electrically connected to a first ring electrode (112) of the one or more ring electrodes (112), wherein the first rubber member (320) comprises a first electrode wire hole (730) through which the first electrode wire (855) extends, or
wherein the first rubber member (320) comprises a potting hole (740) configured to accommodate injection of a potting adhesive through the potting hole (740) into a lumen defined by the elongate tubular inner layer (210), or
further comprising a metallic braided layer that is longitudinally disposed over the exterior surface of the elongate tubular inner layer (210) and under an interior surface of the first rubber member (320), or
wherein the first rubber member (320) has a durometer equal to or less than Shore A65, or
wherein the first rubber member (320) comprises silicone rubber, or
wherein the first rubber member (320) is chemically treated to impart chemical compatibility with at least one of the elongate tubular inner layer (210) or the elongate outer layer (230).

8. The catheter shaft assembly of any one of claim 1 through claim 6, wherein the elongate tubular inner layer (210) comprises a thermoplastic or thermoplastic elastomer material,
optionally wherein the thermoplastic or thermoplastic elastomer material has a durometer of greater than Shore D40,
further optionally wherein the thermoplastic or thermoplastic elastomer material has a durometer in a range of Shore D60 to D85.

9. The catheter shaft assembly of any one of claim 1 through claim 6, wherein the elongate outer layer (230) comprises a thermoplastic or thermoplastic elastomer material.

10. The catheter shaft assembly of claim 9, wherein the thermoplastic or thermoplastic elastomer material is the same as that of the elongate tubular inner layer (210), or wherein the thermoplastic or thermoplastic elastomer material is different from that of an elongate tubular inner layer (210) and has a durometer of less than Shore D60.

11. A method of manufacturing a catheter shaft assembly, the method comprising:
forming a tubular inner thermoplastic layer (810);
forming a first shaft assembly by placing a first rubber member (320) or in situ forming the first rubber member (320) on an exterior surface of the tubular inner thermoplastic layer (810);
forming a second shaft assembly by placing an outer thermoplastic layer (830) over the first shaft assembly;
forming a bonded catheter shaft by thermal-fusion bonding between the tubular inner thermoplastic layer (810) and the outer thermoplastic layer (830) within the second shaft assembly; and
attaching a first ring electrode (112) to the bonded catheter shaft so that the first ring electrode (112) induces a radial hyperelastic deformation of the first rubber member (320) that inhibits loss of an interference fit between the first ring electrode (112) and the bonded catheter shaft.

12. The method of claim 11, wherein the first rubber member (320) encircles and extends along a first length of the bonded catheter shaft.

13. The method of claim 11, wherein forming the first shaft assembly comprises placing a plurality of discrete rubber members on the exterior surface of the tubular inner thermoplastic layer (810) so that the discrete rubber members are circumferentially distributed and spaced apart along a first length of the tubular inner thermoplastic layer (810), wherein the plurality of discrete rubber members comprises the first rubber member (320),
optionally wherein one or more of the discrete rubber members comprise a protruding portion shaped to enhance coupling of the discrete rubber member with the outer thermoplastic layer (830).

14. The method of claim 11, wherein forming the first shaft assembly comprises applying an adhesive to the exterior surface of the tubular inner thermoplastic layer (810),
wherein the adhesive, when cured, is thermoplastic in nature,
optionally wherein the adhesive comprises a cyanoacrylate adhesive comprising at least one of: a cyanoacrylate monomer or oligomer.

15. The method of any one of claim 11 through claim 14, wherein the thermal-fusion bonding comprises melting and reflowing the tubular inner thermoplastic layer (810) and the outer thermoplastic layer (830) of the second shaft assembly under radial compressive pressure, or
wherein forming the bonded catheter shaft further comprises placing a heat-shrinkable tube over the outer thermoplastic layer (830) prior to heating the second shaft assembly, or
wherein the first rubber member (320) has a durometer equal to or less than Shore A65, or
wherein the first rubber member (320) is made of a silicone rubber compound, or
wherein attaching the first ring electrode (112) to the bonded catheter shaft comprises swagging the first ring electrode (112) onto the bonded catheter shaft using a swaging die, or
further comprising treating the first rubber member (320) with one or more silane coupling agent to impart chemical compatibility with at least one of the tubular inner thermoplastic layer (810) or the outer thermoplastic layer (830).

## Patentansprüche

1. Katheterschaftbaugruppe, die Folgendes umfasst:
eine längliche röhrenförmige Innenschicht (210);
ein erstes Gummielement (320), das auf einer Außenoberfläche der länglichen röhrenförmigen inneren Schicht (210) angeordnet ist;
eine längliche äußere Schicht (230), die eine Außenoberfläche des ersten Gummielements (320) oder die längliche röhrenförmige innere Schicht (210) bedeckt; und
eine oder mehrere Ringelektroden (112), die an der länglichen äußeren Schicht (230) oder dem ersten Gummielement (320) befestigt sind und diese umschließen, wobei jede der Ringelektroden (112) eine radiale hyperelastische Verformung des ersten Gummielements (320) bewirkt, die den Verlust einer Presspassung zwischen der Ringelektrode (112) und der länglichen äußeren Schicht (230) oder dem ersten Gummielement (320) verhindert.

2. Katheterschaftbaugruppe nach Anspruch 1, wobei die längliche röhrenförmige innere Schicht (210) die gleiche Länge wie die längliche äußere Schicht (230) aufweist oder wobei das erste Gummielement (320) länglich ist und eine Länge aufweist, die gleich oder kürzer als die der länglichen röhrenförmigen inneren Schicht (210) oder der länglichen äußeren Schicht (230) ist; und wobei das erste Gummielement (320) zwischen der länglichen röhrenförmigen inneren Schicht (210) und der länglichen äußeren Schicht (230) angeordnet ist.

3. Katheterschaftbaugruppe nach Anspruch 1, ferner umfassend ein zweites Gummielement (321);
wobei jedes des ersten Gummielements (320) und des zweiten Gummielements (321) eine Länge aufweist, die gleich oder größer ist als eine Ringelektrode (112) der einen oder der mehreren Ringelektroden (112); und
wobei das erste Gummielement (320) entlang der länglichen röhrenförmigen inneren Schicht (210) längs von dem zweiten Gummielement (321) beabstandet ist, optional ferner ein drittes Gummielement (322) mit einer Länge umfasst, die gleich oder länger als eine Ringelektrode (112) der einen oder der mehreren Ringelektroden (112) ist,
wobei das erste (320), das zweite (321) und das dritte (322) Gummielement längs in gleichen Abständen oder unterschiedlichen Abständen voneinander beabstandet sind.

4. Katheterschaftbaugruppe nach Anspruch 1, wobei das erste Gummielement (320) die längliche röhrenförmige innere Schicht (210) umschließt und sich entlang einer ersten Länge dieser erstreckt.

5. Katheterschaftbaugruppe nach Anspruch 4, ferner umfassend ein zweites Gummielement (321) und ein drittes Gummielement (322),
wobei das erste Gummielement (320), das zweite Gummielement (321) und das dritte Gummielement (322) in Umfangsrichtung entlang der länglichen röhrenförmigen inneren Schicht (210) verteilt und voneinander beabstandet sind; und
wobei jede der einen oder der mehreren Ringelektroden (112) eine radiale hyperelastische Verformung an jedem des ersten Gummielements (320), des zweiten Gummielements (321) und des dritten Gummielements (322) bewirkt, die den Verlust der Presspassung zwischen jeder der Ringelektroden (112) und der länglichen äußeren Schicht (230), dem ersten Gummielement (320), dem zweiten Gummielement (321) oder dem dritten Gummielement (322) verhindert.

6. Katheterschaftbaugruppe nach Anspruch 5,
wobei wenigstens eines des ersten Gummielements (320) und des zweiten Gummielements (321) einen vorstehenden Abschnitt umfasst, der geformt ist, um die Kopplung mit der länglichen äußeren Schicht (230) zu verbessern, oder
ferner umfassend ein viertes Gummielement (423), ein fünftes Gummielement (424) und ein sechstes Gummielement (425), wobei:
das erste Gummielement (320), das zweite Gummielement (321), das dritte Gummielement (322), das vierte Gummielement (423), das fünfte Gummielement (424) und das sechste Gummielement (425) in Umfangsrichtung entlang der ersten Länge der länglichen, röhrenförmigen inneren Schicht (210) angeordnet und voneinander beabstandet sind; und
jede der einen oder der mehreren Ringelektroden (112) eine radiale hyperelastische Verformung jedes des ersten Gummielements (320), des zweiten Gummielements (321), des dritten Gummielements (322), des vierten Gummielements (423), des fünften Gummielements (424) und des sechsten Gummielements (425) bewirkt, die den Verlust der Presspassung zwischen der einen oder den mehreren Ringelektroden (112) und der länglichen äußeren Schicht (230), dem ersten Gummielement (320), dem zweiten Gummielement (321), dem dritten Gummielement (322), dem vierten Gummielement (423), dem fünften Gummielement (424) oder dem sechsten Gummielement (425) verhindert.

7. Katheterschaftbaugruppe nach einem der Ansprüche 1 bis einschließlich 6, wobei das erste Gummielement (320) ein röhrenförmiges Gummielement ist, das wenigstens einen längsorientierten Durchgangsschlitz (225) oder Löcher (227) für die Erleichterung der durch Schmelzfluss induzierten Raumfüllung und der thermischen Verschmelzungsverbindung der länglichen röhrenförmigen inneren Schicht (210), der länglichen äußeren Schicht (230) und des ersten Gummielements (320) umfasst, oder wobei das erste Gummielement (320) einen vorstehenden Abschnitt umfasst, der geformt ist, um die Kopplung mit der länglichen äußeren Schicht (230) oder der länglichen röhrenförmigen inneren Schicht (210) zu verbessern, oder
ferner umfassend einen ersten Elektrodendraht (855), der elektrisch mit einer ersten Ringelektrode (112) der einen oder der mehreren Ringelektroden (112) verbunden ist, wobei das erste Gummielement (320) ein erstes Elektrodendrahtloch (730) umfasst, durch das sich der erste Elektrodendraht (855) erstreckt, oder
wobei das erste Gummielement (320) ein Vergussloch (740) umfasst, das dazu konfiguriert ist, das Einspritzen eines Vergussklebstoffs durch das Vergussloch (740) in ein Lumen aufzunehmen, das durch die längliche röhrenförmige innere Schicht (210) definiert wird, oder
ferner umfassend eine metallische Geflechtschicht, die längs über der Außenoberfläche der länglichen röhrenförmigen inneren Schicht (210) und unter einer Innenoberfläche des ersten Gummielements (320) angeordnet ist, oder
wobei das erste Gummielement (320) eine Shore-Härte von gleich oder weniger als Shore A65 aufweist, oder
wobei das erste Gummielement (320) Silikonkautschuk umfasst, oder
wobei das erste Gummielement (320) chemisch behandelt wird, um eine chemische Kompatibilität mit wenigstens einer der länglichen röhrenförmigen inneren Schicht (210) oder der länglichen äußeren Schicht (230) zu verleihen.

8. Katheterschaftbaugruppe nach einem der Ansprüche 1 bis einschließlich 6, wobei die längliche röhrenförmige innere Schicht (210) ein thermoplastisches oder thermoplastisches Elastomermaterial umfasst,
optional wobei das thermoplastische oder thermoplastische Elastomermaterial eine Shore-Härte von mehr als Shore D40 aufweist,
ferner optional, wobei das thermoplastische oder thermoplastische Elastomermaterial eine Shore-Härte in einem Bereich von D60 bis D85 aufweist.

9. Katheterschaftbaugruppe nach einem der Ansprüche 1 bis einschließlich 6, wobei die längliche äußere Schicht (230) ein thermoplastisches oder thermoplastisches Elastomermaterial umfasst.

10. Katheterschaftbaugruppe nach Anspruch 9, wobei das thermoplastische oder thermoplastische Elastomermaterial dasselbe wie das der länglichen röhrenförmigen innere Schicht (210) ist, oder wobei das thermoplastische oder thermoplastische Elastomermaterial sich von dem einer länglichen röhrenförmigen inneren Schicht (210) unterscheidet und eine Shore-Härte von weniger als D60 aufweist.

11. Verfahren zum Herstellen einer Katheterschaftbaugruppe, wobei das Verfahren Folgendes umfasst:
Ausbilden einer röhrenförmigen thermoplastischen inneren Schicht (810);
Ausbilden einer ersten Schaftbaugruppe, indem ein erstes Gummielement (320) auf einer Außenoberfläche der rohrförmigen thermoplastischen inneren Schicht (810) platziert oder in situ das erste Gummielement (320) darauf ausgebildet wird;
Ausbilden einer zweiten Schaftbaugruppe, indem eine thermoplastischen äußeren Schicht (830) über die erste Schaftbaugruppe platziert wird;
Ausbilden eines verbundenen Katheterschafts durch thermisches Verschmelzungsverbinden der röhrenförmigen thermoplastischen inneren Schicht (810) und der thermoplastischen äußeren Schicht (830) innerhalb der zweiten Schaftbaugruppe; und
Anbringen einer ersten Ringelektrode (112) an den verbundenen Katheterschaft, sodass die erste Ringelektrode (112) eine radiale hyperelastische Verformung des ersten Gummielements (320) bewirkt, die den Verlust einer Presspassung zwischen der ersten Ringelektrode (112) und dem verbundenen Katheterschaft verhindert.

12. Verfahren nach Anspruch 11, wobei das erste Gummielement (320) den verbundenen Katheterschaft entlang einer ersten Länge umschließt und sich entlang dieser erstreckt.

13. Verfahren nach Anspruch 11, wobei das Ausbilden der ersten Schaftbaugruppe das Platzieren einer Mehrzahl von einzelnen Gummielementen auf der Außenoberfläche der röhrenförmigen thermoplastischen inneren Schicht (810) umfasst, sodass die einzelnen Gummielemente in Umfangsrichtung verteilt und entlang einer ersten Länge der röhrenförmigen thermoplastischen inneren Schicht (810) voneinander beabstandet sind, wobei die Mehrzahl von einzelnen Gummielementen das erste Gummielement (320) umfasst,
optional wobei eines oder mehrere der einzelnen Gummielemente einen vorstehenden Abschnitt umfasst/umfassen, der geformt ist, um die Kopplung des einzelnen Gummielements mit der thermoplastischen äußeren Schicht (830) zu verbessern.

14. Verfahren nach Anspruch 11, wobei das Ausbilden der ersten Schaftbaugruppe das Anwenden eines Klebstoffs auf die Außenoberfläche der röhrenförmigen thermoplastischen inneren Schicht (810) umfasst,
wobei der Klebstoff, wenn er ausgehärtet ist, thermoplastischer Natur ist,
optional wobei der Klebstoff ein Cyanacrylatklebstoff umfasst, der wenigstens eines von Folgendem umfasst: einem Cyanacrylatmonomer oder -oligomer.

15. Verfahren nach einem der Ansprüche 11 bis einschließlich 14, wobei das thermische Verschmelzungsverbinden das Schmelzen und Aufschmelzen der röhrenförmigen thermoplastischen inneren Schicht (810) und der thermoplastischen äußeren Schicht (830) der zweiten Schaftbaugruppe unter radialem Pressdruck umfasst, oder
wobei das Ausbilden des verbundenen Katheterschafts ferner das Platzieren eines wärmeschrumpfenden Schlauchs über die thermoplastische äußere Schicht (830) vor dem Erhitzen der zweiten Schaftbaugruppe umfasst, oder
wobei das erste Gummielement (320) eine Shore-Härte von gleich oder weniger als Shore A65 aufweist, oder
wobei das erste Gummielement (320) aus einer Silikonkautschukverbindung hergestellt ist, oder
wobei das Anbringen der ersten Ringelektrode (112) an den verbundenen Katheterschaft das Aufhämmern der ersten Ringelektrode (112) auf den verbundenen Katheterschaft unter Verwendung eines Schmiedesattels umfasst, oder
ferner umfassend das Behandeln des ersten Gummielements (320) mit einem oder mehreren Silan-Haftvermittlern, um eine chemische Kompatibilität mit wenigstens einer der röhrenförmigen thermoplastischen inneren Schicht (810) oder der thermoplastischen äußeren Schicht (830) zu verleihen.

## Revendications

1. Ensemble tige de cathéter comprenant :
une couche intérieure tubulaire allongée (210) ;
un premier élément en caoutchouc (320) disposé sur une surface extérieure de la couche intérieure tubulaire allongée (210) ;
une couche extérieure allongée (230) qui couvre une surface extérieure du premier élément en caoutchouc (320) ou de la couche intérieure tubulaire allongée (210) ; et
une ou plusieurs électrodes annulaires (112) fixées à la couche extérieure allongée (230) ou au premier élément en caoutchouc (320) et l'entourant, chacune des électrodes annulaires (112) provoquant une déformation radiale hyperélastique du premier élément en caoutchouc (320) qui empêche la perte d'un ajustement serré entre l'électrode annulaire (112) et la couche extérieure allongée (230) ou le premier élément en caoutchouc (320).

2. Ensemble tige de cathéter selon la revendication 1, dans lequel la couche intérieure tubulaire allongée (210) a la même longueur que la couche extérieure allongée (230), ou dans lequel le premier élément en caoutchouc (320) est allongé, et a une longueur égale ou inférieure à celle de la couche intérieure tubulaire allongée (210) ou de la couche extérieure allongée (230) ; et dans lequel le premier élément en caoutchouc (320) est disposé entre la couche intérieure tubulaire allongée (210) et la couche extérieure allongée (230).

3. Ensemble tige de cathéter selon la revendication 1, comprenant, en outre, un deuxième élément en caoutchouc (321) ;
dans lequel chacun du premier élément en caoutchouc (320) et du deuxième élément en caoutchouc (321) a une longueur égale ou supérieure à celle d'une électrode annulaire (112) parmi les une ou plusieurs électrodes annulaires (112) ; et
dans lequel le premier élément en caoutchouc (320) est espacé longitudinalement du deuxième élément en caoutchouc (321) le long de la couche intérieure tubulaire allongée (210), comprenant, en outre, éventuellement un troisième élément en caoutchouc (322) ayant une longueur égale ou supérieure à celle d'une électrode annulaire (112) parmi les une ou plusieurs électrodes annulaires (112),
dans lequel les premier (320), deuxième (321) et troisième (322) éléments en caoutchouc sont espacés longitudinalement les uns des autres par des distances égales ou des distances variables.

4. Ensemble tige de cathéter selon la revendication 1, dans lequel le premier élément en caoutchouc (320) entoure la couche intérieure tubulaire allongée (210) et s'étend le long d'une première longueur de celle-ci.

5. Ensemble tige de cathéter selon la revendication 4, comprenant, en outre, un deuxième élément en caoutchouc (321) et un troisième élément en caoutchouc (322), dans lequel le premier élément en caoutchouc (320), le deuxième élément en caoutchouc (321) et le troisième élément en caoutchouc (322) sont répartis dans la direction circonférentielle et espacés les uns des autres le long de la couche intérieure tubulaire allongée (210) ; et
dans lequel chacune des une ou plusieurs électrodes annulaires (112) provoque une déformation radiale hyperélastique de chacun du premier élément en caoutchouc (320), du deuxième élément en caoutchouc (321) et du troisième élément en caoutchouc (322) qui empêche la perte de l'ajustement serré entre chacune des électrodes annulaires (112) et la couche extérieure allongée (230), le premier élément en caoutchouc (320), le deuxième élément en caoutchouc (321) ou le troisième élément en caoutchouc (322).

6. Ensemble tige de cathéter selon la revendication 5,
dans lequel au moins l'un du premier élément en caoutchouc (320) et du deuxième élément en caoutchouc (321) comprend une partie saillante présentant une forme prévue pour renforcer l'accouplement avec la couche extérieure allongée (230), ou comprenant, en outre, un quatrième élément en caoutchouc (423), un cinquième élément en caoutchouc (424) et un sixième élément en caoutchouc (425), dans lequel :
le premier élément en caoutchouc (320), le deuxième élément en caoutchouc (321), le troisième élément en caoutchouc (322), le quatrième élément en caoutchouc (423), le cinquième élément en caoutchouc (424) et le sixième élément en caoutchouc (425) sont répartis dans la direction circonférentielle et espacés les uns des autres le long de la première longueur de la couche intérieure tubulaire allongée (210) ; et
chacune des une ou plusieurs électrodes annulaires (112) provoque une déformation radiale hyperélastique de chacun du premier élément en caoutchouc (320), du deuxième élément en caoutchouc (321), du troisième élément en caoutchouc (322), du quatrième élément en caoutchouc (423), du cinquième élément en caoutchouc (424) et du sixième élément en caoutchouc (425) qui empêche la perte de l'ajustement serré entre les une ou plusieurs électrodes annulaires (112) et la couche extérieure allongée (230), le premier élément en caoutchouc (320), le deuxième élément en caoutchouc (321), le troisième élément en caoutchouc (322), le quatrième élément en caoutchouc (423), le cinquième élément en caoutchouc (424) ou le sixième élément en caoutchouc (425).

7. Ensemble tige de cathéter selon l'une quelconque des revendications 1 à 6, dans lequel le premier élément en caoutchouc (320) est un élément en caoutchouc tubulaire comprenant au moins une fente traversante (225) orientée longitudinalement ou des trous (227) pour faciliter le remplissage des espaces par écoulement à l'état fondu et le liage par fusion thermique de la couche intérieure tubulaire allongée (210), de la couche extérieure allongée (230) et du premier élément en caoutchouc (320), ou dans lequel le premier élément en caoutchouc (320) comprend une partie saillante présentant une forme prévue pour renforcer l'accouplement avec la couche extérieure
allongée (230) ou la couche intérieure tubulaire allongée (210), ou comprenant, en outre, un premier fil d'électrode (855) relié électriquement à une première électrode annulaire (112) parmi les une ou plusieurs électrodes annulaires (112), dans lequel le premier élément en caoutchouc (320) comprend un premier trou pour fil d'électrode (730) à travers lequel s'étend le premier fil d'électrode (855), ou dans lequel le premier élément en caoutchouc (320) comprend un trou pour enrobage (740) conçu pour permettre l'injection d'un adhésif d'enrobage à travers le trou pour enrobage (740) dans une lumière définie par la couche intérieure tubulaire allongée (210), ou
comprenant, en outre, une couche tressée métallique qui est disposée longitudinalement sur la surface extérieure de la couche intérieure tubulaire allongée (210) et sous une surface intérieure du premier élément en caoutchouc (320), ou
dans lequel le premier élément en caoutchouc (320) présente une dureté mesurée au duromètre égale ou inférieure à Shore A65, ou
dans lequel le premier élément en caoutchouc (320) comprend un caoutchouc de silicone, ou
dans lequel le premier élément en caoutchouc (320) est traité chimiquement pour lui conférer une compatibilité chimique avec au moins l'une de la couche intérieure tubulaire allongée (210) et de la couche extérieure allongée (230).

8. Ensemble tige de cathéter selon l'une quelconque des revendications 1 à 6, dans lequel la couche intérieure tubulaire allongée (210) comprend un matériau thermoplastique ou élastomère thermoplastique,
éventuellement, dans lequel le matériau thermoplastique ou élastomère thermoplastique présente une dureté mesurée au duromètre supérieure à Shore D40, éventuellement encore, dans lequel le matériau thermoplastique ou élastomère thermoplastique présente une dureté mesurée au duromètre comprise entre Shore D60 et D85.

9. Ensemble tige de cathéter selon l'une quelconque des revendications 1 à 6, dans lequel la couche extérieure allongée (230) comprend un matériau thermoplastique ou élastomère thermoplastique.

10. Ensemble tige de cathéter selon la revendication 9, dans lequel le matériau thermoplastique ou élastomère thermoplastique est le même que celui de la couche intérieure tubulaire allongée (210), ou dans lequel le matériau thermoplastique ou élastomère thermoplastique est différent de celui de la couche intérieure tubulaire allongée (210) et présente une dureté mesurée au duromètre inférieure à Shore D60.

11. Procédé de fabrication d'un ensemble tige de cathéter, le procédé comprenant :
former une couche thermoplastique intérieure tubulaire (810) ;
former un premier ensemble tige en disposant un premier élément en caoutchouc (320) ou en formant *in situ* le premier élément en caoutchouc (320) sur une surface extérieure de la couche thermoplastique intérieure tubulaire (810) ;
former un second ensemble tige en disposant une couche thermoplastique extérieure (830) sur le premier ensemble tige ;
former une tige de cathéter liée par liage par fusion thermique entre la couche thermoplastique intérieure tubulaire (810) et la couche thermoplastique extérieure (830) à l'intérieur du second ensemble tige ; et
fixer une première électrode annulaire (112) à la tige de cathéter liée de telle sorte que la première électrode annulaire (112) provoque une déformation radiale hyperélastique du premier élément en caoutchouc (320) qui empêche la perte d'un ajustement serré entre la première électrode annulaire (112) et la tige de cathéter liée.

12. Procédé selon la revendication 11, dans lequel le premier élément en caoutchouc (320) entoure la tige de cathéter liée et s'étend le long d'une première longueur de celle-ci.

13. Procédé selon la revendication 11, dans lequel la formation du premier ensemble tige comprend la disposition d'une pluralité d'éléments en caoutchouc individuels sur la surface extérieure de la couche thermoplastique intérieure tubulaire (810) de telle sorte que les éléments en caoutchouc individuels soient répartis dans la direction circonférentielle et espacés les uns des autres le long d'une première longueur de la couche thermoplastique intérieure tubulaire (810), dans lequel la pluralité d'éléments en caoutchouc individuels comprend le premier élément en caoutchouc (320), éventuellement dans lequel un ou plusieurs des éléments en caoutchouc individuels comprennent une partie saillante présentant une forme prévue pour renforcer l'accouplement de l'élément en caoutchouc individuel avec la couche thermoplastique extérieure (830).

14. Procédé selon la revendication 11, dans lequel la formation du premier ensemble tige comprend l'application d'un adhésif à la surface extérieure de la couche thermoplastique intérieure tubulaire (810),
dans lequel l'adhésif, une fois durci, est de nature thermoplastique,
éventuellement dans lequel l'adhésif comprend un adhésif de cyanoacrylate comprenant : un monomère et/ou un oligomère de cyanoacrylate.

15. Procédé selon l'une quelconque des revendications 11 à 14, dans lequel le liage par fusion thermique comprend la fusion et la remise en écoulement de la couche thermoplastique intérieure tubulaire (810) et la couche thermoplastique extérieure (830) du second ensemble tige sous pression de compression radiale, ou
dans lequel la formation de la tige de cathéter liée comprend, en outre, la disposition d'un tube thermorétractable sur la couche thermoplastique extérieure (830) avant de chauffer le second ensemble tige, ou
dans lequel le premier élément en caoutchouc (320) présente une dureté mesurée au duromètre égale ou inférieure à Shore A65, ou
dans lequel le premier élément en caoutchouc (320) est fait d'un mélange de caoutchouc de silicone, ou
dans lequel la fixation de la première électrode annulaire (112) à la tige de cathéter liée comprend le sertissage de la première électrode annulaire (112) sur la tige de cathéter liée au moyen d'un outil de sertissage, ou
comprenant, en outre, le traitement du premier élément en caoutchouc (320) avec un ou plusieurs agents de couplage de silane afin de lui conférer une compatibilité chimique avec au moins l'une de la couche thermoplastique intérieure tubulaire (810) et de la couche thermoplastique extérieure (830).
